# EUROPEAN PATENT APPLICATION

(11) **EP 4 261 225 A1**
(43) Date of publication of application: **18.10.2023**
(21) Application number: 21903911.2
(22) Date of filing: 10.12.2021
(51) Int. Cl.: C07K 16/28, A61K 39/395, A61K 39/00

(54) **ANTI-PD-1 ANTIBODY AND USES THEREOF**

(30) Priority: 10.12.2020 US 202063123705 P
(71) Applicant: Eutilex Co., Ltd., Seoul 08591 (KR)
(72) Inventor: KWON, Byoung S., Gwangmyeong-si Gyeonggi-do 14256 (KR); HWANG, Sun Hee, Goyang-si Gyeonggi-do 10391 (KR); SON, Hyun Tae, Yongin-si Gyeonggi-do 16825 (KR); PARK, Hyeok Jun, Seoul 08584 (KR); YOO, Eun Hye, Seoul 08586 (KR)
(74) Representative: Cabinet Becker et Associés
(86) International application number: PCT/KR2021/018799
(87) International publication number: WO 2022/124866

(57) **Abstract**

The present invention relates to a programmed cell death protein 1 (anti-PD-1) antibody or an antigen-binding fragment thereof; a nucleic acid encoding same; a recombinant expression vector containing the nucleic acid; host cells transfected with the recombinant expression vector; a method for preparing the antibody or the antigen-binding fragment thereof; a bispecific or multispecific antibody comprising the antibody or the antigen-binding fragment thereof; an immune cell engaging bispecific or multispecific antibody comprising at least one scFv of the antibody and at least one scFv of an antibody binding to an immune cell activating antigen; an antibody-drug conjugate (ADC) in which the antibody or the antigen-binding fragment thereof is bound to a drug; a chimeric antigen receptor (CAR) comprising the scFv of the anti-PD-1 antibody as an antigen-binding site of an extracellular domain; immune cells into which the chimeric antigen receptor is introduced; a composition for combination therapy comprising the immune cells; a composition for combination therapy comprising the antibody or the antigen-binding fragment thereof; a composition for the treatment of cancer; and a method for treating cancer.

## Description

### [Technical Field]

The present invention relates to a programmed cell death protein 1 (anti-PD-1) antibody or an antigen-binding fragment thereof; a nucleic acid encoding same; a recombinant expression vector containing the nucleic acid; host cells transfected with the recombinant expression vector; a method for preparing the antibody or the antigen-binding fragment thereof; a bispecific or multispecific antibody including the antibody or the antigen-binding fragment thereof; an immune cell engaging bispecific or multispecific antibody including scFv of the antibody and at least one of scFv of an antibody binding to an immune cell activating antigen; an antibody-drug conjugate (ADC) in which the antibody or the antigen-binding fragment thereof is bound to a drug; a chimeric antigen receptor (CAR) including the scFv of the anti-PD-1 antibody as an antigen-binding site of an extracellular domain; immune cells into which the chimeric antigen receptor is introduced; a composition for combination therapy including the immune cells; a composition for combination therapy including the antibody or the antigen-binding fragment thereof; a composition for the treatment of cancer; and a method for treating cancer.

### [Background Art]

PD-1 is recognized to play an important role in immune regulation and the maintenance of peripheral tolerance. A programmed cell death receptor 1 (PD-1; programmed cell death receptor 1, programmed cell death protein 1, CD279) as an immunosuppressive receptor mainly expressed on activated T and B cells is a member of the immunoglobulin superfamily related to CD28 and cytotoxic T-lymphocyte associated protein-4 (CTLA-4, CD152), and the PD-1 is a type I transmembrane glycoprotein that contains an extracellular Ig variable-type (V-type) that binds to a ligand and a cytoplasmic tail that binds to signaling molecules. The PD-1 is moderately expressed on T, B, and NKT cells and up-regulated by T/B cell receptor signaling on lymphocytes, monocytes, and myeloid cells (Sharpe et al., The function of programmed cell death 1 and its ligands in regulating autoimmunity and infection. Nature Immunology (2007); 8:239-245).

The PD-1 attenuates T-cell responses when bound by PD-L1 (CD274, B7-H1) and/or PD-L2 (CD273, B7-DC). The binding of these ligands to the PD-1 transmits signals that inhibit T-cell proliferation, cytokine production, and cytolytic functions. Blockade of PD-L1 binding to PD-1 enhances tumor-specific CD8+ T-cell immunity, thereby facilitating the removal of tumor cells by an immune system.

The ligands for PD-1, PD-L1 (CD274, B7-H1) and/or PD-L2 (CD273, B7-DC), are expressed in human cancers arising in various tissues. For example, in large sample sets of ovarian cancer, renal cancer, colorectal cancer, pancreatic cancer, liver cancer, and melanoma, PD-L1 expression correlates with poor prognosis and reduces overall survival regardless of subsequent treatment.

PD-L1 expressing tumor cells interact with PD-1 expressing T cells to attenuate T cell activation and evade immune surveillance, thereby contributing to an impaired immune response against the tumor.

Keytruda (ingredient name: pembrolizumab) developed by Merck Co., Ltd. or Opdivo (ingredient name: nivolumab) from BMS Merck Co., Ltd. are commercially available as PD-1 inhibitory antibodies. However, these drugs are effective when they activate T lymphocytes, which attack tumor cells to kill cancer, but have a problem in that the activation degree is slightly expressed. Therefore, there is a need to develop a novel anti-PD-1 antibody that is distinguished from existing antibodies for activating T lymphocytes enough to be effective in inhibiting tumor or cancer growth.

Under this technical background, the present inventors have made intensive efforts to develop a novel anti-PD-1 antibody, confirmed excellent characteristics, efficacy, and the like of the antibody, confirmed that the antibody may be used for the desired cancer treatment, and then completed the present invention.

### [Disclosure]

### [Technical Problem]

An object of the present invention is to provide a novel antibody against programmed cell death protein 1 (PD-1) or an antigen-binding fragment thereof.

Another object of the present invention is to provide a nucleic acid encoding the antibody or the antigen-binding fragment thereof.

Yet another object of the present invention is to provide a recombinant expression vector containing the nucleic acid and host cells transfected with the recombinant expression vector.

Still another object of the present invention is to provide a method for preparing an antibody or an antigen-binding fragment thereof specifically binding to PD-1.

Still yet another object of the present invention is to provide a bispecific or multispecific antibody including the antibody or the antigen-binding fragment thereof.

Still yet another object of the present invention is to provide an immune cell engaging bispecific or multispecific antibody including the antibody or the antigen-binding fragment thereof.

Still yet another object of the present invention is to provide an antibody-drug conjugate (ADC) in which the antibody or the antigen-binding fragment thereof binds to a drug.

Still yet another object of the present invention is to provide a chimeric antigen receptor (CAR) including scFv of the anti-PD-1 antibody as an antigen-binding site of an extracellular domain, immune cells into which the chimeric antigen receptor is introduced, and a composition for combination therapy including the immune cells.

Still yet another object of the present invention is to provide a composition for combination therapy including the antibody or the antigen-binding fragment thereof or the immune cell engaging bispecific or multispecific antibody.

Still yet another object of the present invention is to provide an antibody or an antigen-binding fragment thereof, a bispecific or multispecific antibody including the antibody or the antigen-binding fragment thereof, an antibody-drug conjugate including the antibody or the antigen-binding fragment thereof, a chimeric antigen receptor including the antibody or the antigen-binding fragment thereof, a composition for the treatment of cancer including the chimeric antigen receptor, or a method for treating cancer.

### [Technical Solution]

In order to achieve the object, an aspect of the present invention provides an antibody or an antigen-binding fragment thereof that specifically binds to PD-1, including:
a heavy chain CDR1 including at least one amino acid sequence selected from the group consisting of SEQ ID NOs: 1, 8, and 14;
a heavy chain CDR2 including at least one amino acid sequence selected from the group consisting of SEQ ID NOs: 2, 9, and 15;
a heavy chain CDR3 including at least one amino acid sequence selected from the group consisting of SEQ ID NOs: 3, 4, 10, 16, and 56;
a light chain CDR1 including at least one amino acid sequence selected from the group consisting of SEQ ID NOs: 5, 11, 17, 57, and 58;
a light chain CDR2 including at least one amino acid sequence selected from the group consisting of SEQ ID NOs: 6, 12, and 18; and
a light chain CDR3 including at least one amino acid sequence selected from the group consisting of SEQ ID NOs: 7, 13, and 19.

Another aspect of the present invention provides a nucleic acid encoding the antibody or the antigen-binding fragment thereof.

Yet another aspect of the present invention provides a recombinant expression vector including the nucleic acid.

Still yet another aspect of the present invention provides host cells transfected with the recombinant expression vector.

Still yet another aspect of the present invention provides a method for preparing an antibody or an antigen-binding fragment thereof specifically binding to PD-1, including culturing host cells to produce antibodies; and isolating and purifying the produced antibodies.

Still yet another aspect of the present invention provides a bispecific or multispecific antibody including the antibody or the antigen-binding fragment thereof.

Still yet another aspect of the present invention provides an immune cell engaging bispecific or multispecific antibody including scFv of the antibody and at least one of scFv of an antibody binding to an immune cell activating antigen.

Still yet another aspect of the present invention provides an antibody-drug conjugate (ADC) in which the antibody or the antigen-binding fragment thereof binds to a drug.

Still yet another aspect of the present invention provides a chimeric antigen receptor (CAR) including an extracellular domain including an antigen-binding site, a transmembrane domain, and an intracellular signaling domain, in which the antigen-binding site of the extracellular domain is scFv of the antibody.

Still yet another aspect of the present invention provides immune cells including the chimeric antigen receptor (CAR).

Still yet another aspect of the present invention provides a composition for combination therapy including the immune cells and a drug other than an anti-PD-1 antibody.

Still yet another aspect of the present invention provides a composition for combination therapy including the antibody or the antigen-binding fragment thereof and at least one selected from the group consisting of:
(i) immune cells;
(ii) immune cells comprising an antigen receptor (CAR) containing an scFv fragment for an antibody other than an anti-PD-1 antibody as an extracellular domain; and
(iii) an immune checkpoint inhibitor.

Still yet another aspect of the present invention provides a composition for combination therapy including the bispecific or multispecific antibody and at least one selected from the group consisting of:
(i) immune cells;
(ii) immune cells comprising an antigen receptor (CAR) containing an scFv fragment for an antibody other than an anti-PD-1 antibody as an extracellular domain; and
(iii) an immune checkpoint inhibitor.

Still yet another aspect of the present invention provides a composition for the treatment of cancer including the antibody or the antigen-binding fragment thereof, the bispecific or multispecific antibody including the antibody or the antigen-binding fragment thereof, the antibody-drug conjugate including the antibody or the antigen-binding fragment thereof, the chimeric antigen receptor including the antibody or the antigen-binding fragment thereof, or the immune cells into which the chimeric antigen receptor is introduced.

Still yet another aspect of the present invention provides a method for treating cancer including administering the antibody or the antigen-binding fragment thereof, the bispecific or multispecific antibody including the antibody or the antigen-binding fragment thereof, the antibody-drug conjugate, the chimeric antigen receptor, the immune cells including the chimeric antigen receptor, or a bispecific immune cell conjugate.

### [Advantageous Effects]

According to the present invention, the anti-PD-1 antibody or the antigen-binding fragment thereof exhibits excellent binding affinity to PD-1 and can be usefully used for the prevention or treatment of desired tumors or cancers.

### [Description of Drawings]

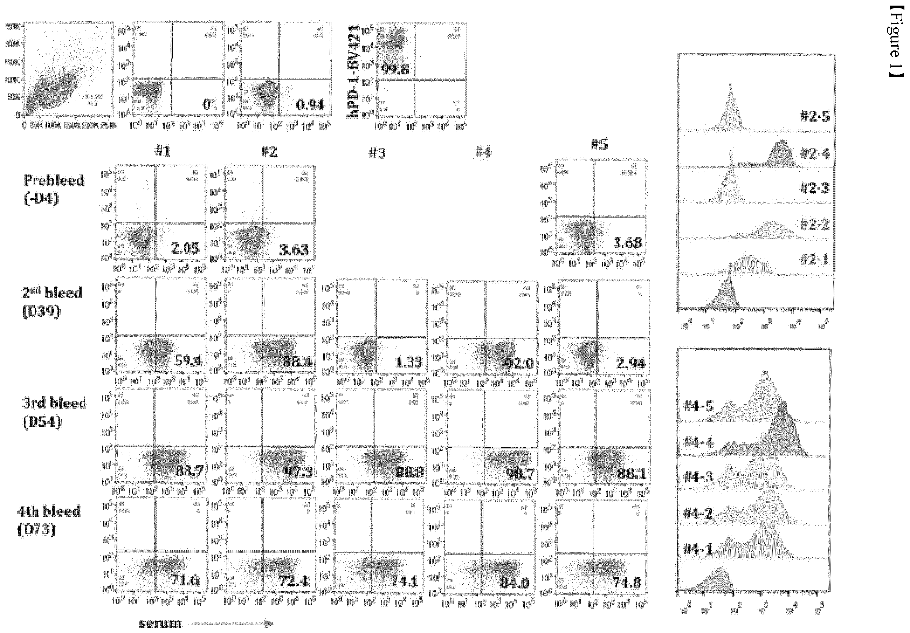

FIG. 1 shows the screening of immunized mice. During immunization of cDNA and rhprotein, the serum of each mouse was isolated to confirm PD-1 antibody formation. It was confirmed that a PD-1 antibody was formed in #2, 4 mice after twice cDNA vaccination, and the PD-1 antibody was formed in all mice after protein boosting. Hybidoma development was performed by fusion using spleens of #2 and 4 mice.

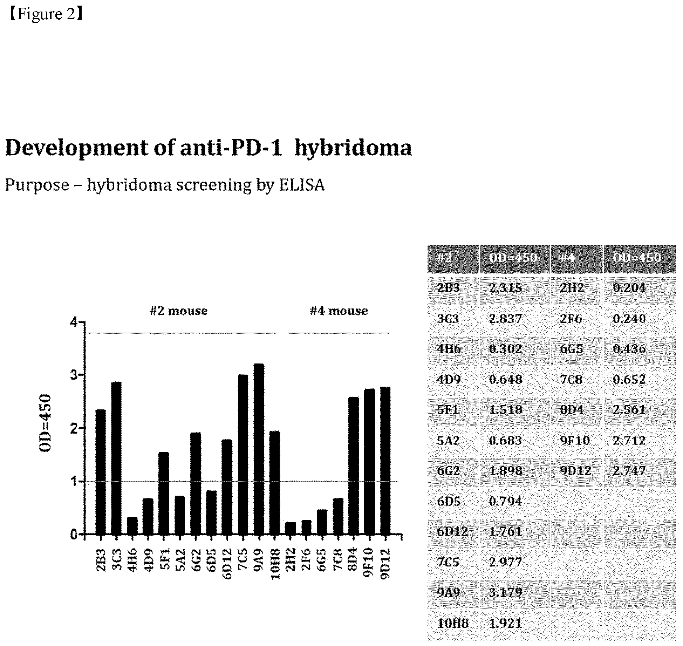

FIG. 2 shows hybridoma screening by ELISA. After about 2 weeks of fusion, sufficient hybridoma clones were formed, and ELISA screening was performed using supernatants. As a result, 12 clones from the fusion of #2 mouse and 7 clones from the fusion of #4 mouse, a total of 19 positive clones were selected, of which 11 clones had an OD = 450 value of 1.0 or higher.

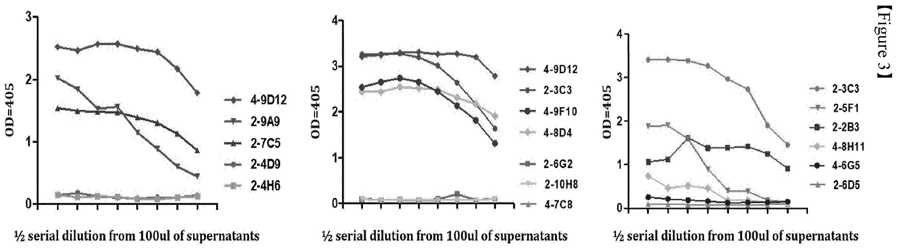

FIG. 3 shows hPD-1 antibody hybridoma screening by Q-ELISA. Each clone was selected by culturing 1 × 10⁶ hybridoma for 24 hr and then quantitatively comparing antibody production by ELISA using the supernatant. As a result, 9 clones of 2-2B3, 2-3C3, 2-5F1, 2-7C5, 2-9A9, 4-8D4, 4-8H11, 4-9D12, and 4-9F10 were selected.

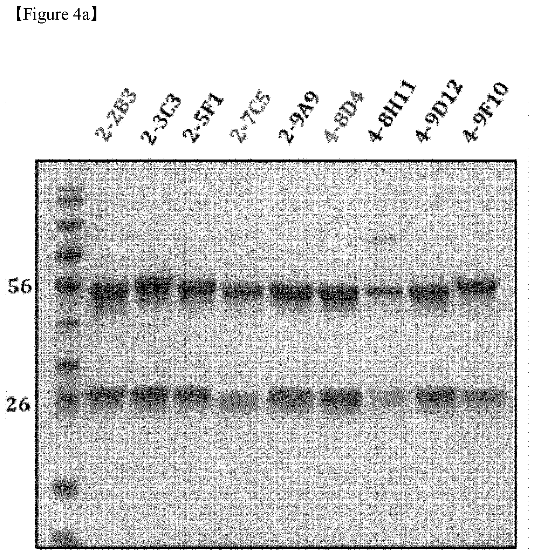

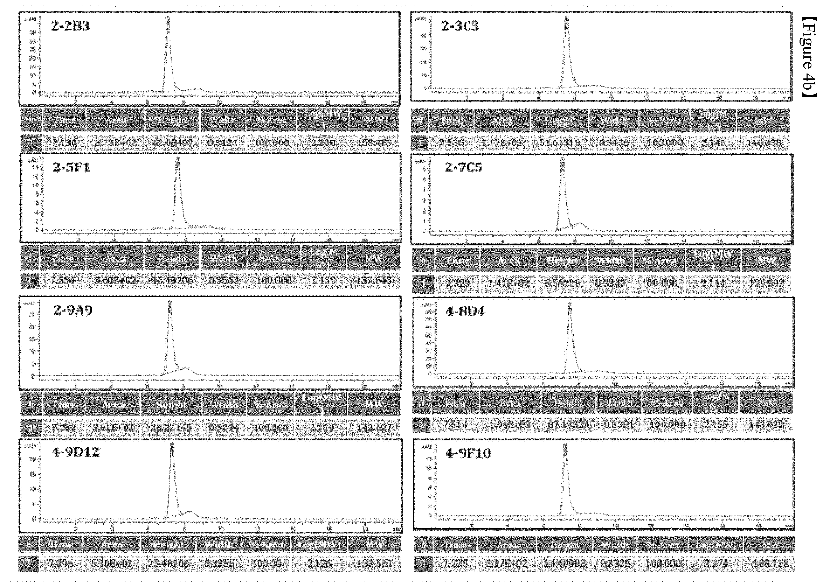

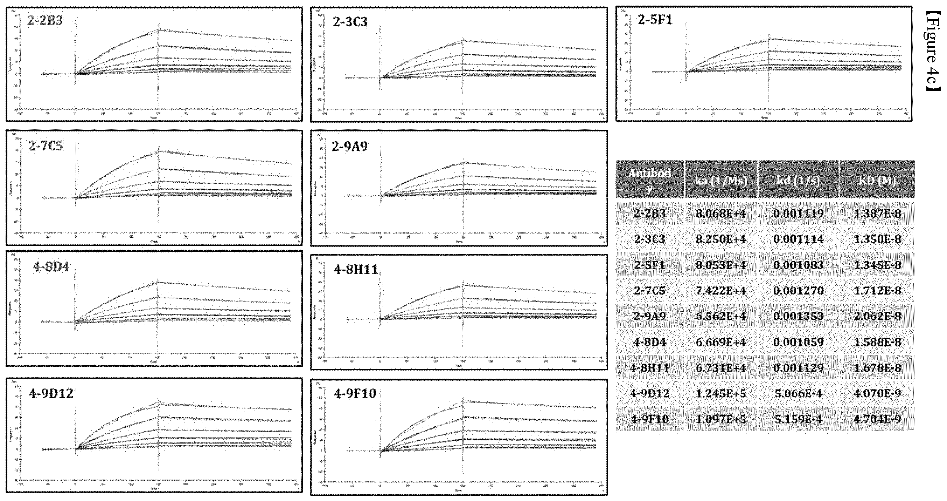

FIGS. 4A to 4C show the characteristics of hPD-1 hybridoma antibodies. The characteristics of purified antibodies were analyzed by SDS-PAGE stain (FIG. 4A), SEC-HPLC (FIG. 4B), and SPR (FIG. 4C). 8 clones showed similar binding affinity to a recombinant human PD-1-His protein (ECD), and among the clones, 4-9D12 and 4-9F10 had slightly higher binding affinity.

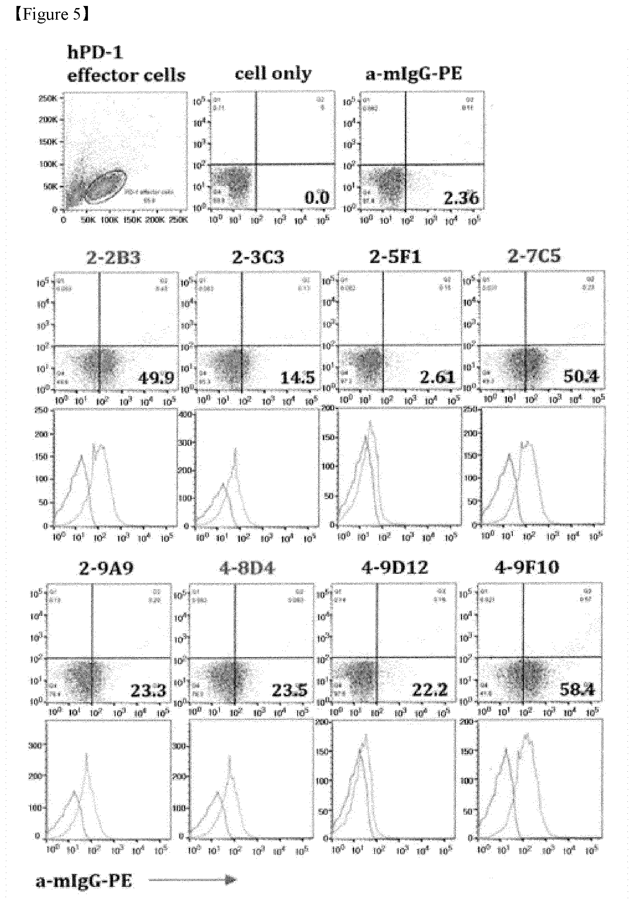

FIG. 5 shows a binding test between hPD-1 antibodies and hPD-1 expressing cells by flow cytometry. As a result of flow cytometry analysis of a binding effect of antibodies to PD-1 using some of the PD-1 expressing effector cells used in PD-1/PD-L1 blocking assay, 2-2B3 (49.9%), 2-7C5 (50.4%), and 4-8D4 (23.5%) were confirmed.

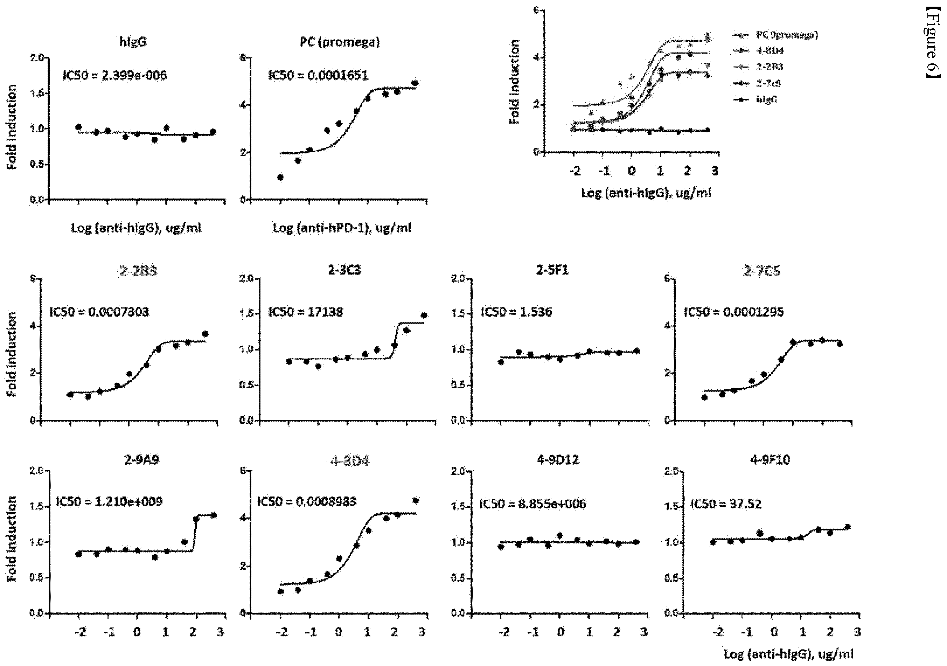

FIG. 6 shows hPD-1/PD-L1 blocking functional assay for hPD-1 hybridoma antibodies. Analysis showed that when two genetically engineered cell lines, PD-1 effector cells and PD-L1 aAPC cells were co-cultured, the PD-1/PD-L1 interaction suppressed TCR-mediated luminescence. On the other hand, when the PD-1/PD-L1 interaction was disrupted by the function of the selected antibody, luminescence was induced by TCR activation. As a result of confirming the blocking effect by 2-fold serial dilution of the antibody selected in the PD-1/PD-L1 co-culture from 5 µg, three clones, 2-2B3, 2-7C5, and 4-8D4 antibodies, showed a similar blocking effect to a control anti-PD-1 antibody.

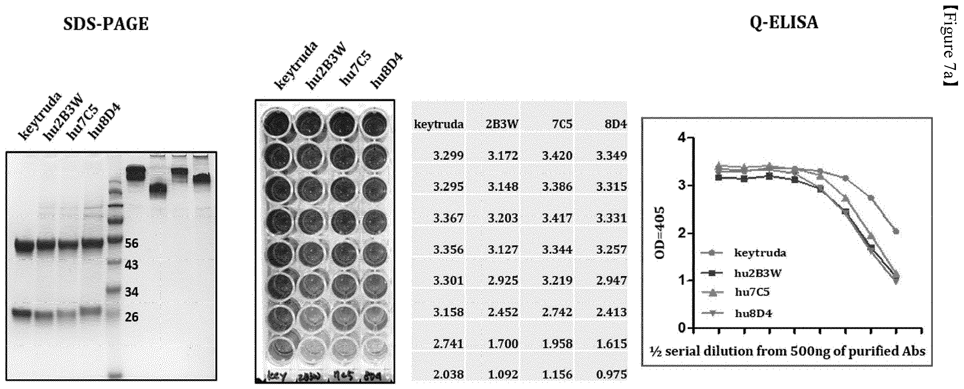

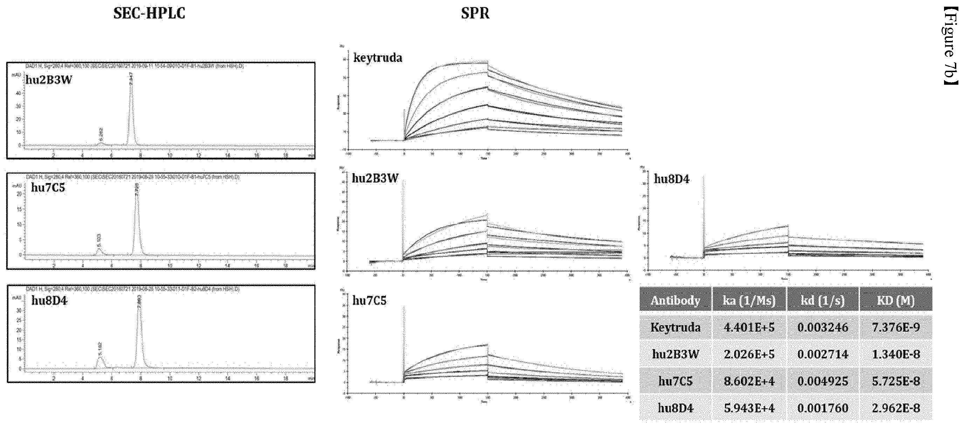

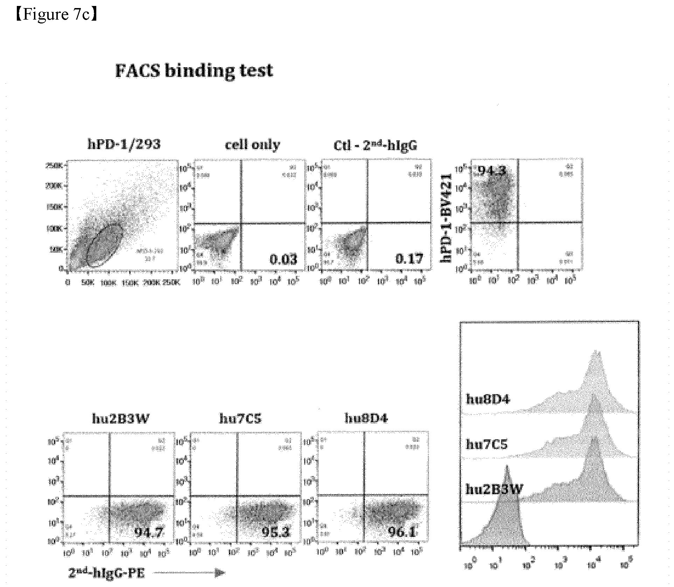

FIGS. 7A to 7C show the humanization of selected 3 clones antibodies. Compared to Keytruda, the characteristics of the antibodies were analyzed, and the binding affinity of the selected antibodies was about 2 to 8 times lower. SDS-PAGE & Q-ELISA (FIG. 7A), SEC-HPLC & SPR (FIG. 7B), and binding effect results (FIG. 7C) of the humanized PD-1 antibodies in hPD-1 expressing were shown.

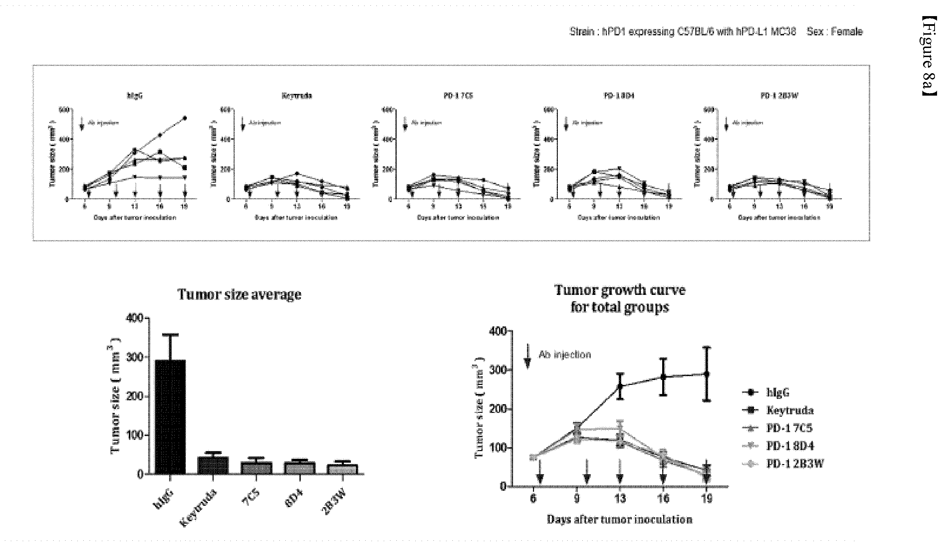

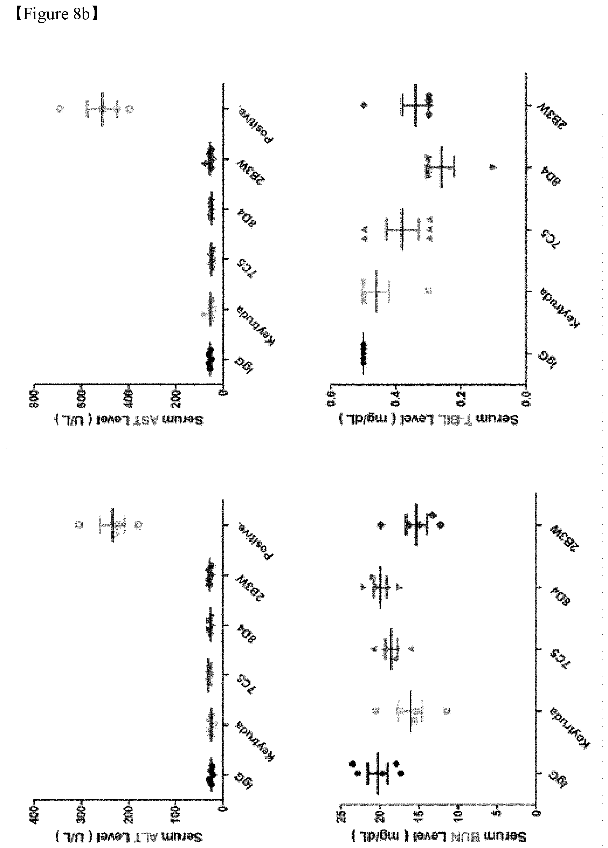

FIG. 8A shows results of analyzing tumor sizes for *in vivo* efficacy analysis of humanized candidate antibodies of the present invention. FIG. 8B shows results of a hepatotoxicity test of the humanized candidate antibodies of the present invention.

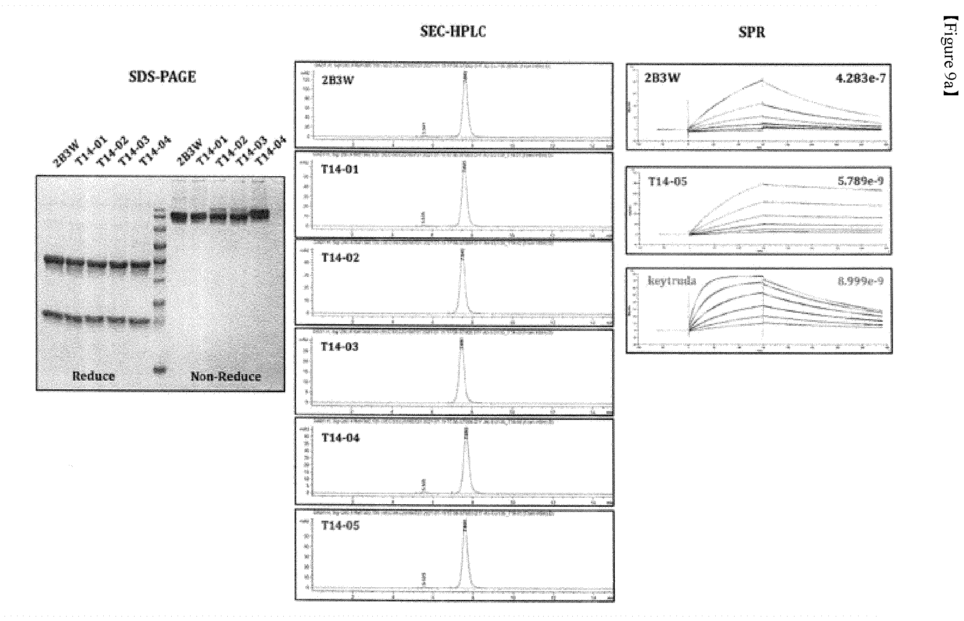

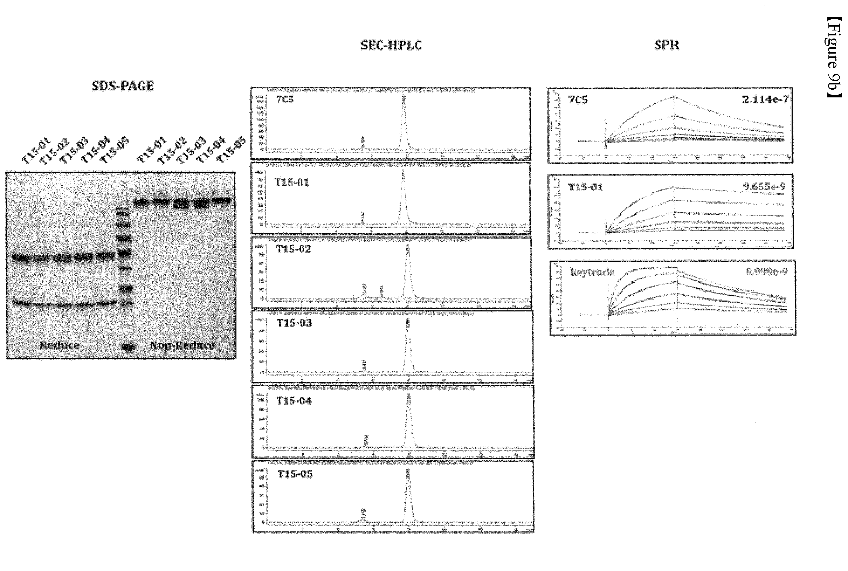

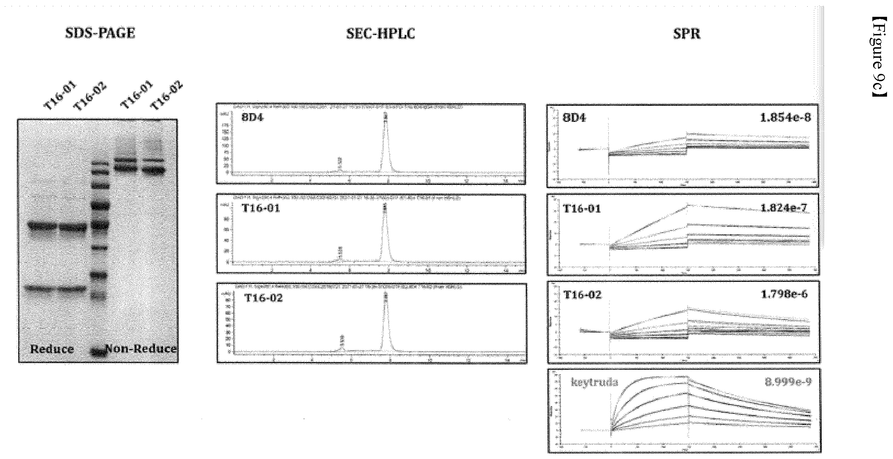

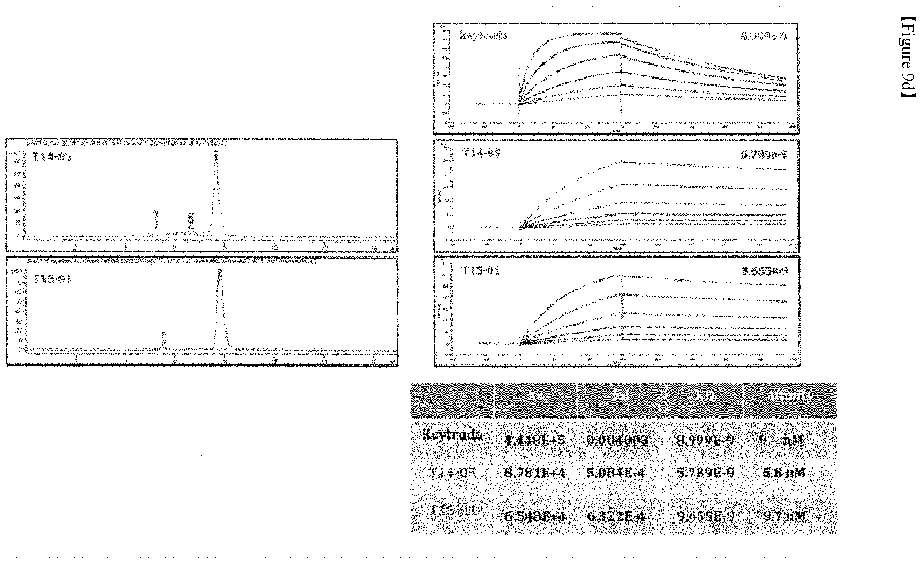

FIGS. 9A to 9C show results of gene engineering to increase the affinity of each candidate antibody to an antigen. FIG. 9A shows a result of screening antibodies of 5 clones with improved affinity of 2B3W, FIG. 9B shows a result of screening antibodies of 5 clones with improved affinity of 7C5, FIG. 9C shows a result of screening antibodies of 2 clones with improved affinity of 8D4, and FIG. 9D shows a result of screening T14-05 (5.8 nM) antibody, which has higher affinity and T15-01 (9.7 nM) antibody, which has similar affinity, compared to the competitor antibody Keytruda (9 nM).

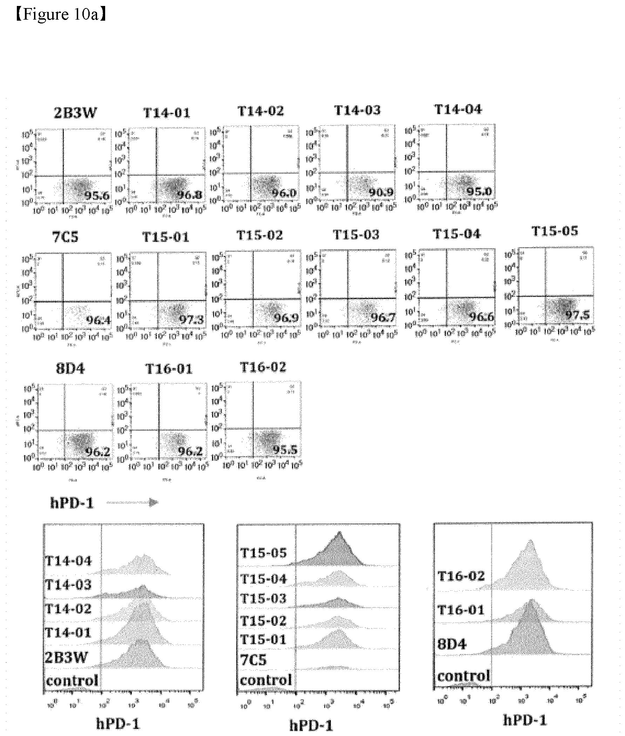

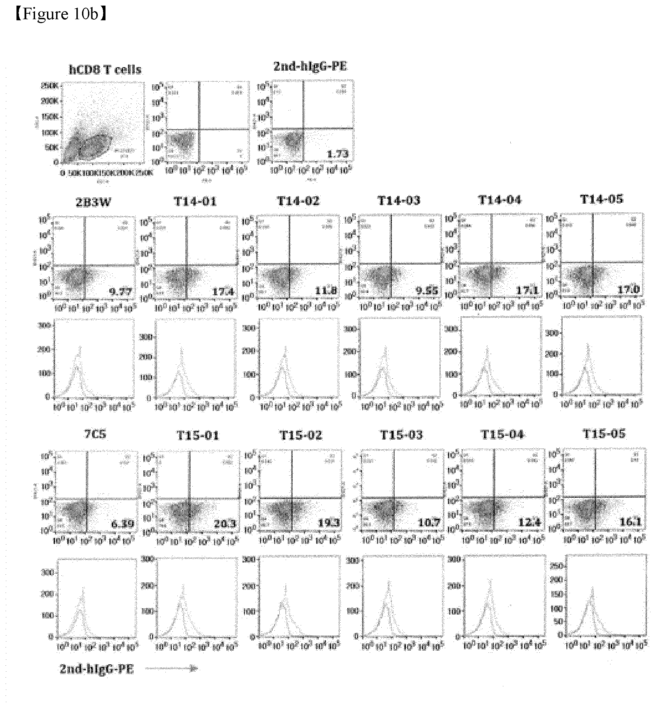

FIG. 10A shows results of analyzing the binding affinity of antibodies selected for affinity improvement of each candidate antibody to cells overexpressing hPD-1 by flow cytometry. FIG. 10B shows results of analyzing the binding affinity of candidate antibodies to activated hCD8 T cells isolated from human blood by flow cytometry.

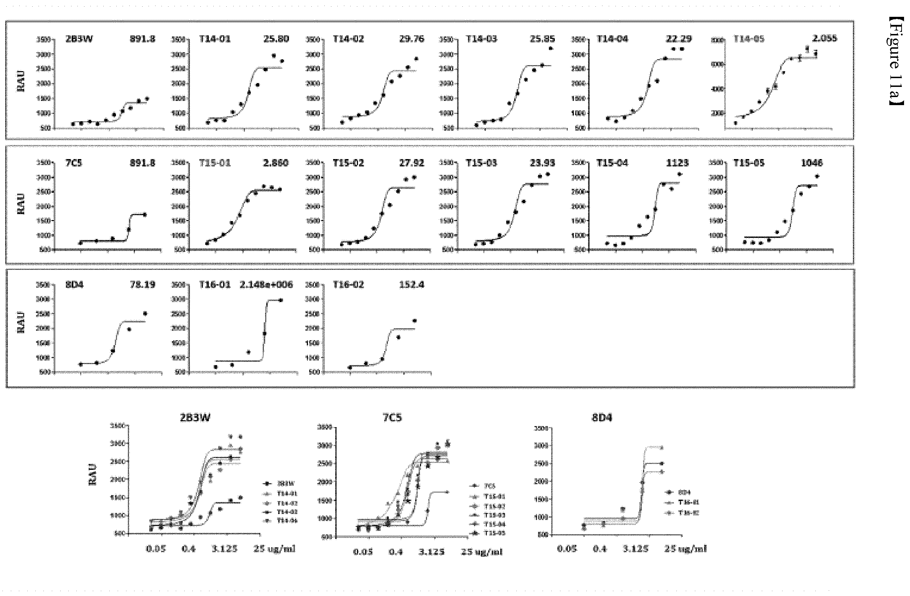

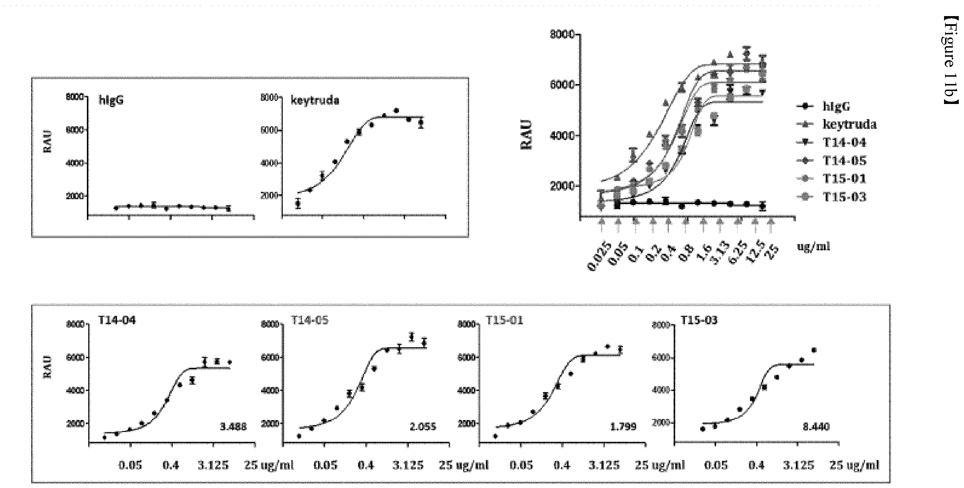

FIG. 11A shows results of analyzing the efficacy capable of inhibiting the binding between effector cells expressing hPD-1 of each candidate antibody and APC cells expressing its receptor hPD-L1. FIG. 11B shows results of comparing T14-05 and T15-01 antibodies with keytruda, a positive control.

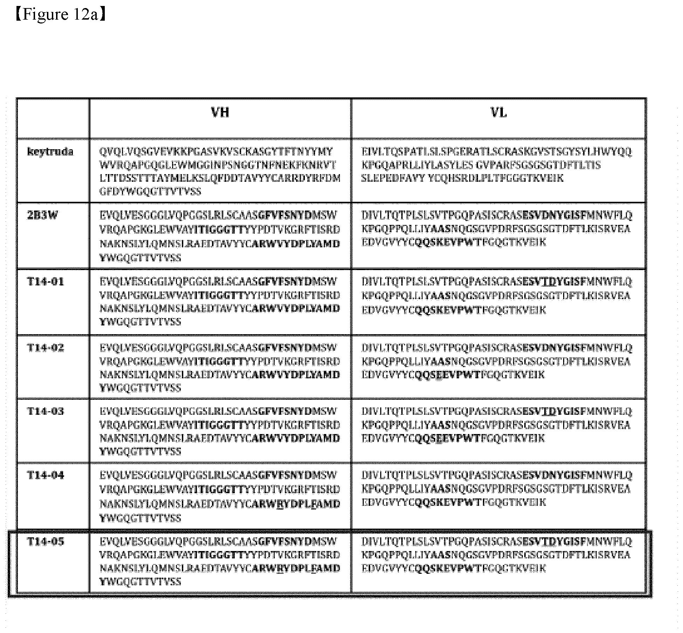

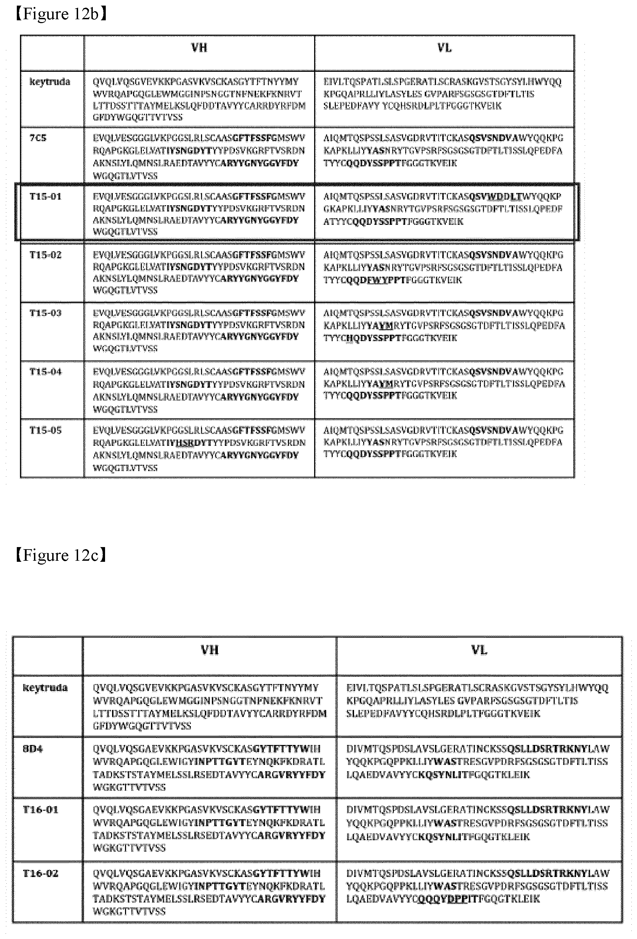

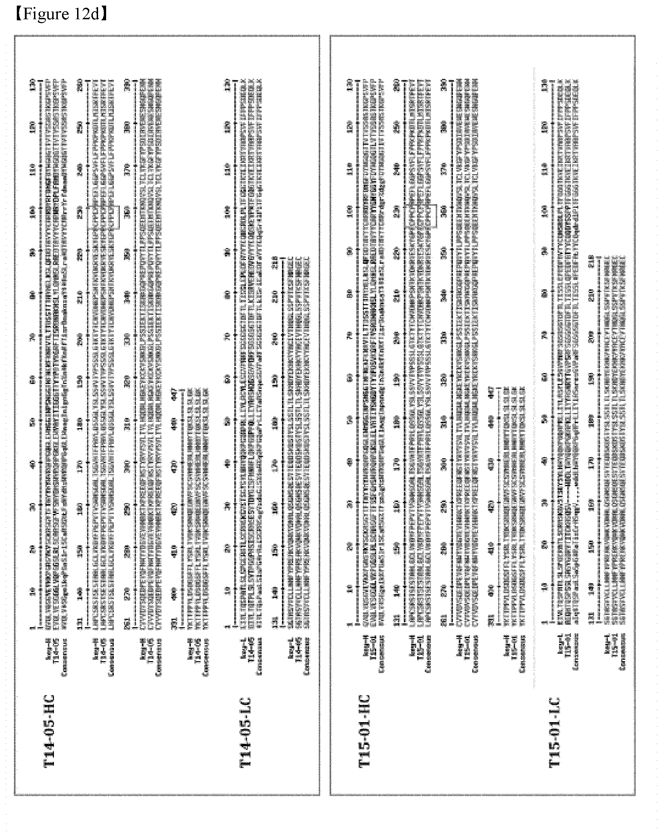

FIG. 12A shows results of sequencing VH, VL, and CDR regions of a selected 2B3W candidate antibody and engineered clone antibodies thereof. FIG. 12B shows results of sequencing VH, VL, and CDR regions of a selected 7C5 candidate antibody and engineered clone antibodies thereof. FIG. 12C shows results of sequencing VH, VL, and CDR regions of a selected 8D4 candidate antibody and engineered clone antibodies thereof. FIG. 12D shows results of comparing sequences of selected final candidate antibodies with an amino acid sequence of keytruda, a positive control.

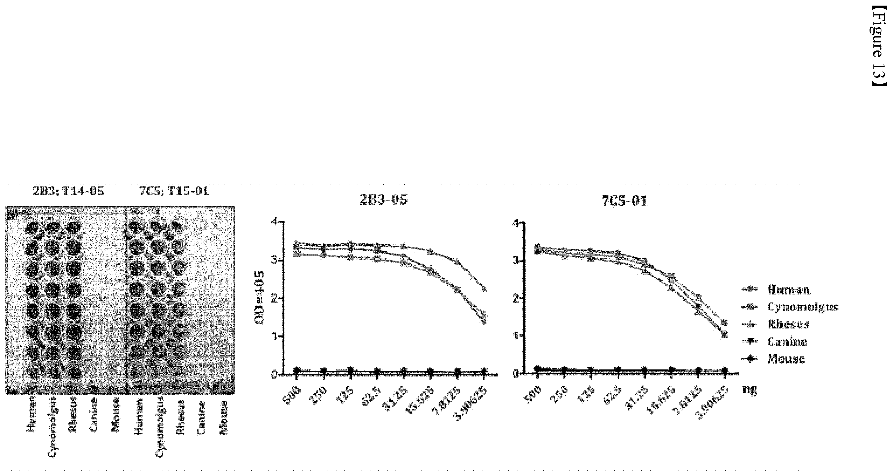

FIG. 13 shows results of interspecificacross-reactivity analysis of selected final candidate antibodies.

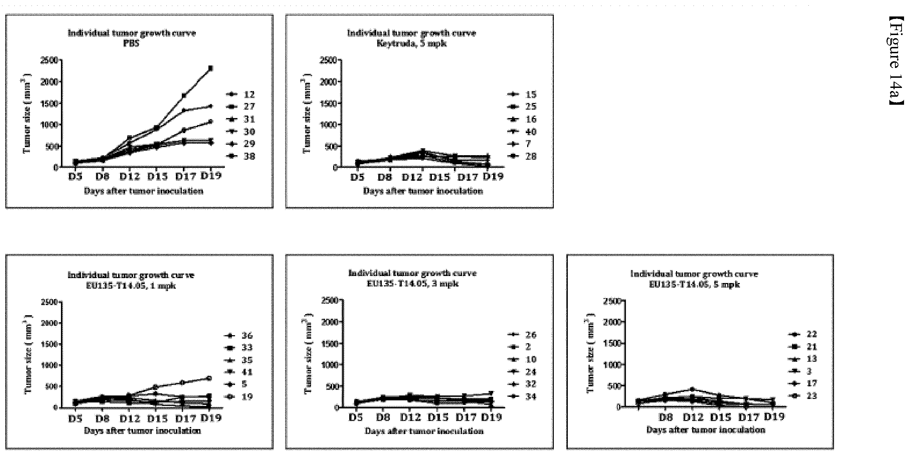

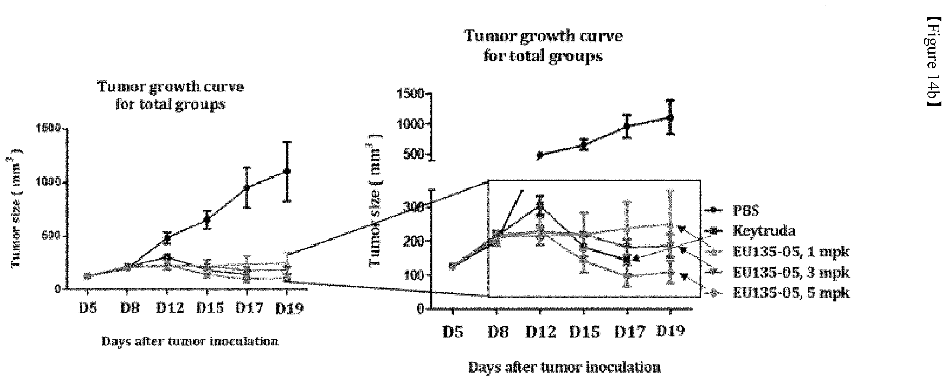

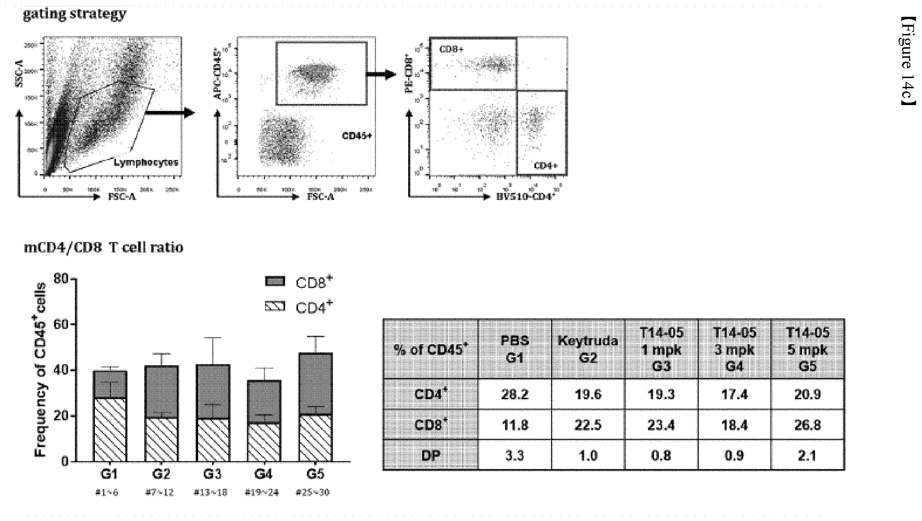

FIGS. 14A to 14C show results of *in vivo* efficacy analysis of final candidate antibodies. FIG. 14A shows tumor size analysis results for each individual in each group according to antibody administration. FIG. 14B shows results of graphing an average value of tumor size in each group. FIG. 14C shows distribution results of CD4 and CD8 T cells in each group.

### [Modes of the Invention]

Unless defined otherwise, all technical and scientific terms used in the present specification have the same meaning as commonly understood by those skilled in the art to which the present invention pertains. In general, the nomenclature used herein is well-known and commonly used in the art.

### Anti-PD-1 antibody

Balb/c mice were immunized by vaccination with a full-length human PD-1 gene and boosting with a recombinant human PD-1 protein, and then the spleen was fused with mouse myeloma cells and sp2/o. Hybridomas producing an anti-hPD-1 antibody were screened and clones were selected. The selected clones were purified to analyze characteristics thereof, and hybridoma antibodies having a function capable of blocking the binding of hPD-1/PD-L1 were selected. Chimeric PD-1 antibodies and finally humanized PD-1 antibodies were developed by cDNA cloning of the selected anti-hPD-1 antibodies.

In an aspect, the present invention relates to an antibody or an antigen-binding fragment thereof specifically binding to PD-1, including: a heavy chain CDR1 including at least one amino acid sequence selected from the group consisting of SEQ ID NOs: 1, 8, and 14; a heavy chain CDR2 including at least one amino acid sequence selected from the group consisting of SEQ ID NOs: 2, 9, and 15; a heavy chain CDR3 including at least one amino acid sequence selected from the group consisting of SEQ ID NOs: 3, 4, 10, 16, and 56; a light chain CDR1 including at least one amino acid sequence selected from the group consisting of SEQ ID NOs: 5, 11, 17, 57, and 58; a light chain CDR2 including at least one amino acid sequence selected from the group consisting of SEQ ID NOs: 6, 12, and 18; and a light chain CDR3 including at least one amino acid sequence selected from the group consisting of SEQ ID NOs: 7, 13, and 19.

The term "antibody" used herein refers to an anti-PD-1 antibody that specifically binds to PD-1. The scope of the present invention includes not only an intact antibody form specifically binding to PD-1, but also an antigen-binding fragment of the antibody molecule.

The intact antibody has a structure having two full-length light chains and two full-length heavy chains, and each light chain is linked to the heavy chain by a disulfide bond.

In the present invention, the term "heavy chain" refers to both a full-length heavy chain and a fragment thereof including a variable region domain VH including an amino acid sequence having a sufficient variable region sequence to impart specificity to the antigen and three constant region domains CH1, CH2, and CH3. In addition, in the present invention, the term "light chain" refers to both a full-length light chain and a fragment thereof including a variable region domain VL including an amino acid sequence having a sufficient variable region sequence to impart specificity to the antigen and a constant region domain CL.

The general antibody includes subtypes of IgA, IgD, IgE, IgM, and IgG, and in particular, IgG includes IgG1, IgG2, IgG3, and IgG4. The heavy chain constant region has gamma (γ), mu (µ), alpha (α), delta (δ), and epsilon (ε) types, and as subclasses, has gamma 1 (γ1), gamma 2 (γ2), gamma 3 (y3), gamma 4 (y4), alpha 1 (α1), and alpha 2 (α2). The light chain constant region has kappa (κ) and lambda (λ) types.

The antigen-binding fragment of the antibody or the antibody fragment refers to a fragment having an antigen-binding function, and includes Fab, F(ab'), F(ab')₂, Fv, and the like. The Fab of the antibody fragments has a structure having variable regions of the light and heavy chains, a constant region of the light chain, and a first constant region (CH1) of the heavy chain, and has one antigen-binding site. The Fab' is different from Fab in that the Fab' has a hinge-region containing one or more cysteine residues at a C-terminal of the heavy chain CH1 domain. The F(ab')₂ is produced when the cysteine residues in the hinge region of Fab' form a disulfide bond.

The Fv refers to the smallest antibody fragment having only a heavy chain variable region and a light chain variable region. A two-chain Fv has a heavy chain variable region and a light chain variable region linked by a non-covalent bond, and a single-chain Fv (scFv) may generally form a dimer-like structure such as two-chain Fv because the variable region of the heavy chain and the variable region of the light chain are covalently linked by a peptide linker or directly linked at a C-terminal. These antibody fragments may be prepared using proteolytic enzymes (for example, Fab may be obtained by restriction cleavage of an intact antibody with papain, and F(ab')₂ may be obtained by cleavage with pepsin), or by genetic recombination technology.

The "Fv" fragment is an antibody fragment that contains an intact antibody recognition and binding site. This region is a dimer in which one heavy chain variable domain and one light chain variable domain are linked.

The "Fab" fragment contains variable and constant domains of the light chain and a variable domain and a first constant domain (CH1) of the heavy chain. The F(ab')₂ antibody fragment generally includes a pair of Fab' fragments covalently linked by cysteines in the hinge region present at the C-terminal of the Fab' fragment.

The "single-chain Fv (scFv)" antibody fragment is a construct consisting of a single polypeptide chain including the VH and VL domains of an antibody. A polypeptide linker may further be included between the VH and VL domains to allow the scFv to form a desired structure for antigen binding.

In an embodiment, the antibody of the present invention is a monoclonal antibody, a multispecific antibody, a human antibody, a humanized antibody, a chimeric antibody, an scFv, an Fab fragment, an F(ab')₂ fragment, a disulfide-bond Fvs (sdFv) and an anti-idiotypic (anti-Id) antibody, or epitope-binding fragments of the antibodies, but is not limited thereto.

The heavy chain constant region may be selected from any one isotype of gamma (γ), mu (µ), alpha (α), delta (δ), or epsilon (ε). For example, the constant region is gamma 1 (IgG1), gamma 2 (IgG2), gamma 3 (IgG3), or gamma 4 (IgG4). The light chain constant region may be a kappa or lambda type.

The monoclonal antibody refers to an antibody obtained from a substantially homogeneous antibody group, i.e., the same thing except for possible naturally occurring mutations in which individual antibodies including the group may be present in a small amount. The monoclonal antibody is highly specific to be induced against a single antigenic site. In contrast to conventional (polyclonal) antibodies, which typically include different antibodies for different determinants (epitopes), each monoclonal antibody is directed against a single determinant on the antigen.

The "epitope" means a protein determinant to which the antibody may specifically bind. The epitope usually consists of a group of chemically active surface molecules, such as amino acids or sugar side chains, and generally has specific 3D structural characteristics as well as specific charge characteristics. Conformational and non-conformational epitopes are distinguished from each other in that the binding to the former is lost, but the binding to the latter is not lost in the presence of a denaturing solvent.

The "humanized" type non-human (e.g., mouse) antibody is a chimeric antibody that contains a minimal sequence derived from a non-human immunoglobulin. In most cases, the humanized antibody is human immunoglobulin (recipient antibody) obtained by replacing residues from a hypervariable region of a recipient with residues from a hypervariable region of a non-human species (donor antibody), such as mouse, rat, rabbit, or non-human primate, which retains the desired specificity, affinity, and capacity.

The "human antibody" is a molecule derived from human immunoglobulin, and means that the entire amino acid sequence constituting an antibody, including a complementarity determining region and a structural region, is composed of human immunoglobulin.

The human antibody includes a fragment of the antibody exhibiting a desired biological activity as well as a "chimeric" antibody (immunoglobulin) in which some of the heavy and/or light chains are identical to or homologous to a corresponding sequence in an antibody derived from a particular species or belonging to a particular antibody class or subclass, while the remaining chain(s) are identical to or homologous to the corresponding sequence in an antibody derived from another species or belonging to another antibody class or subclass.

The "variable region" of the antibody used herein refers to the light and heavy chain portions of an antibody molecule that include an amino acid sequence of a complementarity determining region (CDR; that is, CDR1, CDR2, and CDR3) and a framework region (FR). VH refers to a variable domain of the heavy chain. VL refers to a variable domain of the light chain.

The "Complementarity Determining Region (CDR)" refers to amino acid residues of an antibody variable domain, which is required for antigen binding. Each variable domain typically has three CDR regions identified as CDR1, CDR2, and CDR3 .

The anti-PD-1 antibody or the antigen-binding fragment thereof according to the present invention may include, for example:
(i) a heavy chain variable region including a heavy chain CDR1 of SEQ ID NO: 1, a heavy chain CDR2 of SEQ ID NO: 2, and a heavy chain CDR3 of SEQ ID NO: 3, and a light chain variable region including a light chain CDR1 of SEQ ID NO: 5, a light chain CDR2 of SEQ ID NO: 6, and a light chain CDR3 of SEQ ID NO: 7;
(ii) a heavy chain variable region including a heavy chain CDR1 of SEQ ID NO: 1, a heavy chain CDR2 of SEQ ID NO: 2, and a heavy chain CDR3 of SEQ ID NO: 4, and a light chain variable region including a light chain CDR1 of SEQ ID NO: 5, a light chain CDR2 of SEQ ID NO: 6, and a light chain CDR3 of SEQ ID NO: 7;
(iii) a heavy chain variable region including a heavy chain CDR1 of SEQ ID NO: 8, a heavy chain CDR2 of SEQ ID NO: 9, and a heavy chain CDR3 of SEQ ID NO: 10, and a light chain variable region including a light chain CDR1 of SEQ ID NO: 11, a light chain CDR2 of SEQ ID NO: 12, and a light chain CDR3 of SEQ ID NO: 13;
(iv) a heavy chain variable region including a heavy chain CDR1 of SEQ ID NO: 14, a heavy chain CDR2 of SEQ ID NO: 15, and a heavy chain CDR3 of SEQ ID NO: 16, and a light chain variable region including a light chain CDR1 of SEQ ID NO: 17, a light chain CDR2 of SEQ ID NO: 18, and a light chain CDR3 of SEQ ID NO: 19;
(v) a heavy chain variable region including a heavy chain CDR1 of SEQ ID NO: 1, a heavy chain CDR2 of SEQ ID NO: 2, and a heavy chain CDR3 of SEQ ID NO: 56, and a light chain variable region including a light chain CDR1 of SEQ ID NO: 57, a light chain CDR2 of SEQ ID NO: 6, and a light chain CDR3 of SEQ ID NO: 7; or
(vi) a heavy chain variable region including a heavy chain CDR1 of SEQ ID NO: 8, a heavy chain CDR2 of SEQ ID NO: 9, and a heavy chain CDR3 of SEQ ID NO: 10, and a light chain variable region including a light chain CDR1 of SEQ ID NO: 58, a light chain CDR2 of SEQ ID NO: 12, and a light chain CDR3 of SEQ ID NO: 13.

The "framework region (FR)" is variable domain residues other than CDR residues. Each variable domain typically has four FRs of FR1, FR2, FR3, and FR4.

The binding affinity of the anti-PD-1 antibody to PD-1 is in the range of 10⁻⁵ M to 10⁻¹² M. For example, the binding affinity of the anti-PD-1 antibody to PD-1 is 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M.

The antibody or the antigen-binding fragment thereof that binds to PD-1 may include a heavy chain variable region including at least one amino acid sequence selected from the group consisting of SEQ ID NOs: 20, 21, 23, 25, 27, 28, 30, 32, 48, and 50. In addition, the antibody or the antigen-binding fragment thereof that binds to PD-1 may include a light chain variable region including at least one amino acid sequence selected from the group consisting of SEQ ID NOs: 22, 24, 26, 29, 31, 33, 49, 51, 54, and 55.

In a specific embodiment according to the present invention, the antibody or the antigen-binding fragment thereof may include:
a heavy chain variable region of SEQ ID NO: 20 and a light chain variable region of SEQ ID NO: 22;
a heavy chain variable region of SEQ ID NO: 21 and a light chain variable region of SEQ ID NO: 22;
a heavy chain variable region of SEQ ID NO: 23 and a light chain variable region of SEQ ID NO: 24;
a heavy chain variable region of SEQ ID NO: 25 and a light chain variable region of SEQ ID NO: 26;
a heavy chain variable region of SEQ ID NO: 27 and a light chain variable region of SEQ ID NO: 29;
a heavy chain variable region of SEQ ID NO: 28 and a light chain variable region of SEQ ID NO: 29;
a heavy chain variable region of SEQ ID NO: 30 and a light chain variable region of SEQ ID NO: 31;
a heavy chain variable region of SEQ ID NO: 32 and a light chain variable region of SEQ ID NO: 33;
a heavy chain variable region of SEQ ID NO: 48 and a light chain variable region of SEQ ID NO: 49;
a heavy chain variable region of SEQ ID NO: 50 and a light chain variable region of SEQ ID NO: 51;
a heavy chain variable region of SEQ ID NO: 52 and a light chain variable region of SEQ ID NO: 53; or
a heavy chain variable region of SEQ ID NO: 54 and a light chain variable region of SEQ ID NO: 55. scFv

The scFv is an antibody fragment, as a construct consisting of a single polypeptide chain including the VH and VL domains of an antibody. A polypeptide linker may further be included between the VH and VL domains to allow the scFv to form a desired structure for antigen binding.

In an embodiment, in a single chain Fv (scFv) including the VH and VL domains of the antibody, the VH and VL domains may be linked to each other via a linker. A heavy chain variable region including at least one amino acid sequence selected from the group consisting of SEQ ID NOs: 20, 21, 23, 25, 27, 28, 30, 32, 48, and 50 may be linked to a light chain variable region including at least one amino acid sequence selected from the group consisting of SEQ ID NOs: 22, 24, 26, 29, 31, 33, 49, 51, 54, and 55 via a linker.

In a specific embodiment according to the present invention, the VH and VL domains may include:
a heavy chain variable region of SEQ ID NO: 20 and a light chain variable region of SEQ ID NO: 22;
a heavy chain variable region of SEQ ID NO: 21 and a light chain variable region of SEQ ID NO: 22;
a heavy chain variable region of SEQ ID NO: 23 and a light chain variable region of SEQ ID NO: 24;
a heavy chain variable region of SEQ ID NO: 25 and a light chain variable region of SEQ ID NO: 26;
a heavy chain variable region of SEQ ID NO: 27 and a light chain variable region of SEQ ID NO: 29;
a heavy chain variable region of SEQ ID NO: 28 and a light chain variable region of SEQ ID NO: 29;
a heavy chain variable region of SEQ ID NO: 30 and a light chain variable region of SEQ ID NO: 31;
a heavy chain variable region of SEQ ID NO: 32 and a light chain variable region of SEQ ID NO: 33;
a heavy chain variable region of SEQ ID NO: 48 and a light chain variable region of SEQ ID NO: 49;
a heavy chain variable region of SEQ ID NO: 50 and a light chain variable region of SEQ ID NO: 51;
a heavy chain variable region of SEQ ID NO: 52 and a light chain variable region of SEQ ID NO: 53; or
a heavy chain variable region of SEQ ID NO: 54 and a light chain variable region of SEQ ID NO: 55.

The linker may be a peptide linker and have a length of about 10 to 25 aa. For example, hydrophilic amino acids such as glycine and/or serine may be included, but are not limited thereto.

Specifically, the linker may include, for example, (GS)ₙ, (GGS)ₙ, (GSGGS)ₙ, or (GₙS)ₘ (n and m are each 1 to 10), but the linker may be, for example, (GₙS)ₘ (n and m are each 1 to 10). Specifically, the linker may include GGGGS.

A "phage display" is a technique of displaying a variant polypeptide as a fusion protein with at least a portion of an envelope protein on the surface of a phage, e.g., a filamentous phage particle. The usefulness of the phage display lies in the fact that sequences that bind to a target antigen with high affinity may be rapidly and efficiently sorted by targeting a large library of randomized protein variants. Displaying peptide and protein libraries on the phage has been used to screen millions of polypeptides to determine polypeptides with specific binding properties.

The phage display technology has provided a powerful tool for generating and selecting novel proteins that bind to specific ligands (e.g., antigens). Using phage display technology, large libraries of protein variants may be generated and sequences that bind to the target antigen with high affinity may be rapidly sorted. A nucleic acid encoding a variant polypeptide is fused with a nucleic acid sequence encoding a viral envelope protein, e.g., a gene III protein or a gene VIII protein. A monovalent phage display system has been developed in which a nucleic acid sequence encoding a protein or polypeptide is fused with a nucleic acid sequence encoding a portion of the gene III protein. In the monovalent phage display system, gene fusions are expressed at low levels and a wild-type gene III protein is also expressed to maintain particle infectivity.

Demonstrating the expression of peptides on a filamentous phage surface and the expression of functional antibody fragments in the periplasm of *E. coli* is important for developing antibody phage display libraries. Libraries of antibodies or antigen-binding polypeptides have been prepared in many methods, for example, a method for altering a single gene by inserting a random DNA sequence or a method for cloning related gene series. The library may be screened for the expression of antibodies or antigen-binding proteins with desired characteristics.

The phage display technology has several advantages as compared with conventional hybridoma and recombinant methods for producing antibodies with desired characteristics. This technology enables the generation of a large antibody library with diverse sequences in a short time without using animals. Production of hybridomas or production of humanized antibodies may require several months of production time. In addition, since no immunization is required, the phage antibody library may produce antibodies even against toxic or low-antigenic antigens. The phage antibody library may also be used to produce and identify novel therapeutic antibodies.

Techniques for producing human antibodies from immunized, non-immunized human, germline sequences, or unsensitized B cell Ig repertory may be used using the phage antibody library. A variety of lymphoid tissues may be used to prepare unsensitized or non-immune antigen-binding libraries.

A technique capable of identifying and isolating high-affinity antibodies from the phage display library is important for isolating novel antibodies for treatment. The isolating of the high-affinity antibodies from the library may depend on the size of the library, the efficiency of production in bacterial cells, and the diversity of the library. The size of the library is reduced by inefficient production due to improper folding of the antibody or the antigen-binding protein and the presence of stop codons. The expression in bacterial cells may be inhibited when the antibody or the antigen-binding domain is not properly folded. The expression may be improved by alternating mutations of residues on the surface of the variable/constant interface or the selected CDR residues. The sequence of the framework region is one element for providing proper folding when the antibody phage library is produced in bacterial cells.

In high-affinity antibody isolation, it is important to produce a diverse library of antibodies or antigen-binding proteins. It has been found that CDR3 regions often participate in antigen binding. Since the CDR3 region on the heavy chain varies considerably in size, sequence, and structural conformation, it is possible to produce a variety of libraries using the same.

In addition, diversity may be generated by randomizing the CDR regions of the variable heavy and light chains using all 20 amino acids at each position. When using all 20 amino acids, variant antibody sequences with high diversity may be produced and a chance of identifying novel antibodies may be increased.

The antibody or the antibody fragment of the present invention may include not only the sequence of the anti-PD-1 antibody of the present invention described herein, but also a biological equivalent thereof, within a range capable of specifically recognizing PD-1. For example, additional changes may be made to the amino acid sequence of the antibody to further improve the binding affinity and/or other biological properties of the antibody. Such modifications include, for example, deletion, insertion, and/or substitution of amino acid sequence residues of the antibody. These amino acid variants are made based on the relative similarity of amino acid side-chain substituents, such as hydrophobicity, hydrophilicity, charges, sizes, and the like. By analysis of the size, shape, and type of the amino acid side-chain substituent, it can be seen that arginine, lysine, and histidine are all positively charged residues; alanine, glycine, and serine have similar sizes; and phenylalanine, tryptophan, and tyrosine have similar shapes. Accordingly, based on these considerations, arginine, lysine, and histidine; alanine, glycine, and serine; and phenylalanine, tryptophan, and tyrosine may be biologically functional equivalents.

Considering the variants having the above-described biologically equivalent activity, the antibody or the nucleic acid molecule encoding the antibody of the present invention is interpreted to include a sequence representing substantial identity with the sequence described in SEQ ID NO. The substantial identity means a sequence exhibiting homology of at least 90%, most preferably homology of at least 95%, homology of 96% or more, 97% or more, 98% or more, and 99% or more, when the sequence of the present invention is aligned to correspond to any other sequence as much as possible and the aligned sequence is analyzed using an algorithm commonly used in the art. Alignment methods for sequence comparison are known in the art. An NCBI Basic Local Alignment Search Tool (BLAST) is accessible from NBCI, etc., and may be used in conjunction with sequence analysis programs such as blastp, blasm, blastx, tblastn, and tblastx on the Internet. BLAST is accessible at www.ncbi.nlm.nih.gov/BLAST/. A sequence homology comparison method using these programs can be found at www.ncbi.nlm.nih.gov/BLAST/blast_ help.html.

Based thereon, the antibody or the antigen-binding fragment thereof of the present invention may have homology of 90%, 91%, 92%, 93%, 94%, 95%, 96%, 97%, 98%, 99 %, or 99 % or more as compared with the specified sequence or the entirety described in the specification. Such homology may be determined by sequence comparison and/or alignment by methods known in the art. For example, the percent sequence homology of nucleic acids or proteins according to the present invention may be determined using a sequence comparison algorithm (i.e., BLAST or BLAST 2.0), manual alignment, or visual inspection.

In another aspect, the present invention relates to a nucleic acid encoding the antibody or the antigen-binding fragment thereof. The antibody or the antigen-binding fragment thereof may be recombinantly produced by isolating the nucleic acid encoding the antibody or the antigen-binding fragment thereof of the present invention.

The "nucleic acid" has a meaning of comprehensively including DNA (gDNA and cDNA) and RNA molecules, and nucleotides, which are basic structural units in the nucleic acid, include not only natural nucleotides, but also analogues with modified sugar or base moieties. A sequence of the nucleic acid encoding heavy and light chain variable regions of the present invention may be modified. The modification includes addition, deletion, or non-conservative or conservative substitution of nucleotides.

The nucleic acid encoding the antibody or the antigen-binding fragment thereof binding to PD-1 may include a nucleic acid encoding a heavy chain variable region or light chain variable region selected from the group consisting of SEQ ID NOs: 34 to 47.

In a specific embodiment according to the present invention, the nucleic acid may include:
a nucleic acid encoding a heavy chain variable region of SEQ ID NO: 34 and a nucleic acid encoding a light chain variable region of SEQ ID NO: 36;
a nucleic acid encoding a heavy chain variable region of SEQ ID NO: 35 and a nucleic acid encoding a light chain variable region of SEQ ID NO: 36;
a nucleic acid encoding a heavy chain variable region of SEQ ID NO: 37 and a nucleic acid encoding a light chain variable region of SEQ ID NO: 38;
a nucleic acid encoding a heavy chain variable region of SEQ ID NO: 39 and a nucleic acid encoding a light chain variable region of SEQ ID NO: 40;
a nucleic acid encoding a heavy chain variable region of SEQ ID NO: 41 and a nucleic acid encoding a light chain variable region of SEQ ID NO: 43;
a nucleic acid encoding a heavy chain variable region of SEQ ID NO: 42 and a nucleic acid encoding a light chain variable region of SEQ ID NO: 43;
a nucleic acid encoding a heavy chain variable region of SEQ ID NO: 44 and a nucleic acid encoding a light chain variable region of SEQ ID NO: 45; or
a nucleic acid encoding a heavy chain variable region of SEQ ID NO: 46 and a nucleic acid encoding a light chain variable region of SEQ ID NO: 47.

The DNA encoding the antibody may be easily isolated or synthesized using conventional molecular biological techniques (e.g., by using an oligonucleotide probe capable of specifically binding to DNA encoding the antibody and the heavy and light chains), and the nucleic acid is isolated and inserted into a replicable vector to be additionally cloned (DNA-amplified) or additionally expressed. Based thereon, yet another aspect of the present invention relates to a recombinant expression vector including the nucleic acid.

As used herein, the term "vector" is a means for expressing a target gene in host cells, and includes plasmid vectors, cosmid vectors, bacteriophage vectors, adenoviral vectors, retroviral vectors, viral vectors such as adeno-associated viral vectors, and the like. Components of the vector generally include, but are not limited to, one or more of the following: signal sequences, origins of replication, one or more antibiotic-resistant marker genes, enhancer elements, promoters, and transcription termination sequences. The nucleic acid encoding the antibody is operably linked, such as promoters and transcription termination sequences.

The "operably linked" means a functional linkage between a nucleic acid expression regulatory sequence (e.g., an array of promoter, signal sequence, or transcriptional regulator binding sites) and the other nucleic acid sequence, so that the regulatory sequence regulates the transcription and/or translation of the other nucleic acid sequence.

When a prokaryotic cell is used as a host, the regulatory sequence generally includes strong promoters (e.g., tac promoter, lac promoter, lacUV5 promoter, lpp promoter, pLλ promoter, pRλ promoter, rac5 promoter, amp promoter, recA promoter, SP6 promoter, trp promoter, and T7 promoter, etc.) capable of promoting transcription, ribosome binding sites for initiation of translation, and transcription/translation termination sequences. In addition, for example, when a eukaryotic cell is used as a host, promoters derived from a genome of a mammalian cell (e.g., metallothionein promoter, β-actin promoter, human hemoglobin promoter, and human muscle creatine promoter); or promoters derived from mammalian viruses (e.g., adenovirus late promoter, vaccinia virus 7.5 K promoter, SV40 promoter, cytomegalovirus (CMV) promoter, tk promoter of HSV, mouse mammary tumor virus (MMTV) promoter, LTR promoter of HIV, promoter of Moloney Virus promoter of Epstein-barr virus (EBV), and promoter of Rouse Sarcoma Virus (RSV)) may be used, and usually has a polyadenylation sequence as a transcription termination sequence.

In some cases, the vector may also be fused with other sequences to facilitate purification of antibodies expressed therefrom. The sequences to be fused include, for example, glutathione S-transferase (Pharmacia, USA), maltose binding protein (NEB, USA), FLAG (IBI, USA), 6x His (hexahistidine; Quiagen, USA), and the like.

The vector includes an antibiotic-resistant gene commonly used in the art as a selection marker, and may include resistant genes to, for example, ampicillin, gentamicin, carbenicillin, chloramphenicol, streptomycin, kanamycin, geneticin, neomycin, and tetracycline.

In another aspect, the present invention relates to host cells transfected with the recombinant expression vector. The host cells used to produce the antibody of the present invention may be prokaryotic, yeast, or higher eukaryotic cells, but are not limited thereto.

The host cells may use prokaryotic host cells, such as *Escherichia coli, Bacillus sp.* strains such as *Bacillus subtilus* and *Bacillus thuringiensis, Streptomyces, Pseudomonas* (e.g., *Pseudomonasputida*)*, Proteus mirabilis* and *Staphylococcus* (e.g., *Staphylocus carnosus*)*.*

However, interest in animal cells is greatest, and examples of useful host cell lines may include COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, or HT1080, but are not limited thereto.

In yet another aspect, the present invention relates to a method for preparing an antibody or an antigen-binding fragment thereof specifically binding to PD-1, including culturing the host cells to produce antibodies; and isolating and purifying the produced antibodies.

The host cells may be cultured in various media. Commercially available media may be used as a culture medium without limitation. All other necessary supplements known to those skilled in the art may also be included in suitable concentrations. Culture conditions such as a temperature, a pH, and the like are already used with host cells selected for expression and will be apparent to those skilled in the art.

The antibody or antigen-binding fragment thereof may be recovered by removing impurities by, for example, centrifugation or ultrafiltration, and purifying the result product using, for example, affinity chromatography and the like. Additional other purification techniques may be used, such as anion or cation exchange chromatography, hydrophobic interaction chromatography, hydroxylapatite chromatography, and the like.

### Bispecific or multispecific antibody

In another aspect, the present invention relates to a bispecific or multispecific antibody including the antibody or the antigen-binding fragment thereof.

The bispecific antibody refers to an antibody having binding or antagonistic ability to one or more targets, and refers to a form in which antibodies having binding or antagonistic ability for two different targets are bound, or an antibody having binding affinity to one target and a substance having antagonistic ability to the other target are bound.

The multispecific antibody refers to an antibody having binding specificities for at least three different antigens. The multispecific antibody is a tri-specific or higher antibody, for example, a tri-specific antibody, a tetra-specific antibody, or an antibody that targets four targets or higher.

Antibodies belonging to the bispecific or multispecific antibody may be classified into scFv-based antibodies, Fab-based antibodies, IgG-based antibodies, and the like. Since the bispecific or multispecific antibody may suppress or amplify two or more signals simultaneously, the bispecific or multispecific antibody is more effective than a case of suppressing/amplifying one signal. Compared to a case where each signal is treated with each signal inhibitor, low-dose administration is possible, and two or more signals may be suppressed/amplified at the same time and space.

Methods of preparing the bispecific or multispecific antibody are well known. Traditionally, recombinant production of the bispecific antibody is based on the co-expression of two or more immunoglobulin heavy/light chain pairs, under conditions in which the two or more heavy chains have different specificities.

For bispecific or multispecific antibodies based on scFv, hybrid scFv may be prepared in a heterodimeric form by combining the VLs and VHs of different scFvs, respectively, to create a diabody, and different scFvs may be linked together to produce tendem ScFvs, and CH1 and CL of Fab may be expressed at the end of each scFv to produce a heterodimeric miniantibody. Some amino acids of a CH3 domain, which is a homodimeric domain of Fc, are substituted to be modified into a heterodimeric structure in the form of a `knob into hole', and these modified CH3 domains are expressed at different ends of each scFv to prepare a minibody in the form of heterodimeric scFv.

Fab-based bispecific or multispecific antibodies may be prepared in a heterodimeric Fab form by combining individual Fab' for a specific antigen with each other using disulfide bonds or mediators and may be prepared by expressing scFv for different antigens at the end of the heavy or light chain of specific Fab to have two antigen valencies, or four antigen valencies in a homodimeric form by placing a hinge region between the Fab and scFv. In addition, the Fab-based bispecific or multispecific antibodies may be obtained by chemically conjugating a dual-target bibody having three antigen valencies by fusing scFvs for different antigens to the light chain and heavy chain ends of Fab, a triple-target bibody having three antigen valencies by fusing different scFvs to the light chain and heavy chain ends of Fab, respectively, and different three Fabs.

For IgG-based bispecific or multispecific antibodies, a method for producing bispecific antibodies has been known by Trion Pharma Co., Ltd. by hybridizing mice and rat hybridomas to produce hybrid hybridomas, so-called quadromas. In addition, bispecific antibodies may be prepared in a so-called `Hole and Knob' form, which is produced in a heterodimeric form by modifying some amino acids of the CH3 homodimeric domain of Fc for different heavy chains while sharing the light chain portions. In addition to heterodimeric bispecific antibodies, two different scFvs may be fused and expressed to constant domains instead of the variable domains of the light and heavy chains of IgG, respectively, to produce homodimeric type (scFv)4-IgG. In addition, ImClone Co., Ltd. reported that based on IMC-1C11, a chimeric monoclonal antibody against human VEGFR-2, bispecific antibodies are prepared by fusing only a single variable domain for a mouse Platelet-derived Growth Factor Receptor-α to an amino terminal of the light chain of the antibody. In addition, through a so-called `dock and lock (DNL)' method using a dimerization and docking domain (DDD) of a protein kinase A (PKA) R subunit and an anchoring domain of PKA, the bispecific antibodies may be prepared into antibodies having multiple antigen valencies to CD20.

Very various recombinant antibody formats, for example, bispecific or multispecific antibodies of bivalent or higher, trivalent or higher, or tetravalent or higher, have been developed. For example, the bispecific or multispecific antibodies may also include antibodies of bivalent or higher, trivalent or higher, or tetravalent or higher disclosed in International Patent Publication Nos. WO2001/077342, WO2009/080251, WO2009/080252, WO2009/080253, WO2009/080254, WO2010/112193, WO2010/115589, WO2010/136172, WO2010/145792, WO2010/145793, and WO2011/117330. The antibodies of bivalent or higher, trivalent or higher, or tetravalent or higher indicate that two or more binding domains, three or more binding domains, or four or more binding domains are present in the antibody molecule, respectively.

In a specific embodiment, the bispecific or multispecific antibody according to the present invention may include the anti-PD-1 antibody or the antigen-binding fragment, specifically in the form of an IgG complete antibody or a fragment thereof, such as a single-chain Fv, V_{H} domain and/or V_{L} domain, Fab or (Fab)₂.

In addition, an antibody or an antigen-binding fragment thereof that binds to a different target from the antibody targeting the PD-1 may include one or more selected from the group consisting of, for example, FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1. The antibody or the antigen-binding fragment thereof may be specifically included in the form of a IgG complete antibody or a fragment thereof, for example, a single-chain Fv, V_{H} domain and/or V_{L} domain, Fab or (Fab)₂.

The bispecific or multispecific antibodies according to the present invention may secure additional binding specificity induced or mediated by other targets, in addition to PD-1.

For example, the bispecific antibody according to the present invention may simultaneously target PD-1 and one selected from the group consisting of FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

For example, the multispecific antibody according to the present invention may simultaneously target PD-1 and two or more selected from the group consisting of FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

### Immune cell engaging bispecific or multispecific antibody

In still another aspect, the present invention relates to an immune cell engaging bispecific or multispecific antibody including scFv of the antibody and at least one of scFv of an antibody binding to an immune cell activating antigen.

The immune cell engaging bispecific or multispecific antibody temporarily induces cytolytic synapses between cytotoxic T cells and cancer target cells to release toxins.

In an embodiment, the immune cell activating antigen may be selected from the following examples, and an antibody or an antigen-binding fragment thereof binding thereto may act as an immune cell engager:
a T cell activating antigen such as CD3, TCRα, TCRβ, TCRγ, TCRξ, ICOS, CD28, CD27, HVEM, LIGHT, CD40, 4-1BB, OX40, DR3, GITR, CD30, TIM1, SLAM, CD2, or CD226;
an NK cell activating antigen such as NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160;
a B-cell activating antigen such as OX40, CD40, or CD70;
a macrophage activating antigen such as CD2 agonist, CD40, CD70, Toll-like Receptor (TCR) agonist, CD47, STING, or OX40L; or
a dendritic cell activating antigen such as a CD2 agonist, OX40, OX40L, 41BB agonist, TCR agonist, CD47 agonist, or STING agonist.

The immune cell engager is specifically described in US Patent Publication No. 2017/0368169, which is incorporated herein by reference.

Specifically, the immune cell engaging bispecific or multispecific antibody includes tandem scFv and may bind to the following antigens and surface antigens on cancer cells. The surface antigen on the cancer cells is PD-1, which is targeted by the antibody according to the present invention:
CD3, TCRα, TCRβ, TCRγ, TCRξ, ICOS, CD28, CD27, HVEM, LIGHT, CD40, 4-1BB, OX40, DR3, GITR, CD30, TIM1, SLAM, CD2, or CD226;
NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160;
OX40, CD40, or CD70;
CD2 agonist, CD40, CD70, Toll-like Receptor (TCR) agonist, CD47, STING, or OX40L; or
CD2 agonist, OX40, OX40L, 41BB agonist, TCR agonist, CD47 agonist, or STING agonist.

The immune cell engaging bispecific or multispecific antibody may be a structure of, for example, VL(PD-1)-VH(PD-1)-VH(CD3 or CD16A)-VL(CD3 or CD16A), VH(PD-1)-VL(PD-1)-VH(CD3 or CD16A)-VL(CD3 or CD16A), VH(CD3 or CD16A)-VL(CD3 or CD16A)-VH(PD-1)-VL(PD-1), or VH(CD3 or CD16A)-VL(CD3 or CD16A)-VL(PD-1)-VH(PD-1) form.

The scFv includes a heavy chain variable region including at least one amino acid sequence selected from the group consisting of SEQ ID NOs: 20, 21, 23, 25, 27, 28, 30, 32, 48, and 50 and a light chain variable region including at least one amino acid sequence selected from the group consisting of SEQ ID NOs: 22, 24, 26, 29, 31, 33, 49, 51, 54, and 55, and the heavy chain variable region and the light chain variable region may be linked to each other via a linker.

The linker may be a peptide linker and have a length of about 10 to 25 aa. For example, hydrophilic amino acids such as glycine and/or serine may be included.

The linker may include, for example, (GS)ₙ, (GGS)ₙ, (GSGGS)ₙ, or (GₙS)ₘ (n and m are each 1 to 10), but the linker may be, for example, (GₙS)ₘ (n and m are each 1 to 10). Specifically, the linker may include GGGGS.

Examples of the immune cell engaging bispecific or multispecific antibody include blinatumomab (Amgen) which binds to CD3 and CD19; solitomab (Amgen) which binds to CD3 and EpCAM; MEDI 565 (MedImmune, Amgen) which binds to CD3 and CEA; and BAY2010112 (Bayer, Amgen) which binds to CD3 and PSMA. Exemplary DARTs include MGD006 (Macrogenics) which binds to CD3 and CD123; and MGD007 (Macrogenics) which binds to CD3 and gpA33. Exemplary TandAbs may include AFM11 (Affimed Therapeutics) which binds to CD3 and CD19; and AFM13 (Affimed Therapeutics) which binds to CD30 and CD16A.

### Antibody-drug conjugate (ADC)

In still yet another aspect, the present invention relates to an antibody-drug conjugate (ADC) in which the antibody or the antigen-binding fragment thereof binds to a drug.

In the antibody-drug conjugate, an anti-cancer drug needs to be stably bound to an antibody before delivering the anti-cancer drug to target cancer cells. The drug delivered to the target needs to be released from the antibody to induce the death of the target cells. To this end, the drug needs to have sufficient cytotoxicity to induce the death of the target cells when released from the target cells while stably binding to the antibody.

In one embodiment, the antibody may bind to a drug through a linker. The linker is a site of linking the anti-PD-1 antibody and the drug, and may allow the drug to be released from the antibody in a cleavable form under intracellular conditions, that is, in an intracellular environment, and the antibody is stable during systemic circulation by reflecting a long half-life of the antibody, and the linkage between the linker and the drug should not affect the stability and pharmacokinetics of the antibody.

The linker may include, for example, a cleavable linker or a non-cleavable linker. In the case of the cleavable linker, a peptide linker may be cleaved by intracellular peptidase or protease enzyme, such as lysosomal or endosomal protease, and in the case of the non-cleavable linker, for example, a thioether linker, the drug may be released after the antibody is non-selectively cleaved by intracellular hydrolysis.

In an embodiment, the cleavable linker may include a peptide linker. The peptide linker has a length of at least 2 or more amino acids. For example, a dipeptide of Val-Cit, Val-Ala, or Val-Cit or Phe-Leu or Gly-Phe-Leu-Gly may be included. Examples of linker are specifically described in International Patent Publication No. WO2004/010957, which may be incorporated herein by reference.

The antibody-drug conjugate is encapsulated into cancer cells through an endosome-lysosome pathway after an antibody region of the ADC binds to the antigen of the target cancer cells to form an ADC-antigen complex. In this case, the intracellular release of the cytotoxic drug is regulated by the internal environment of the endosome/lysosome.

In one embodiment, the cleavable linker is pH sensitive and may be sensitive to hydrolysis at a specific pH value. In general, the pH sensitive linker may be hydrolyzed in acidic conditions. For example, acid labile linkers, that may be hydrolyzed in lysosomes, may include hydrazone, semicarbazone, thiosemicarbazone, cis-aconitic amide, orthoester, acetal, ketal, and the like.

In another embodiment, the linker may also be cleaved under reducing conditions, and for example, a disulfide linker may correspond thereto. Various disulfide bonds may be formed by using N-succinimidyl-S-acetylthioacetate (SATA), N-succinimidyl-3-(2-pyridyldithio)propionate (SPDP), N-succinimidyl-3-(2-pyridyldithio)butyrate (SPDB), and N-succinimidyl-oxycarbonyl-alpha-methyl-alpha-(2-pyridyl-dithio)toluene (SMPT). These disulfide linkers may be cleaved by disulfide exchange with thiols of intracellular glutathione.

The drug and/or drug-linker may be randomly conjugated through lysines of the antibody or conjugated through cysteines exposed when the disulfide bond chain is reduced. In some cases, the linker-drug may be linked through a genetically engineered tag, for example, cysteines present in a peptide or protein. The genetically engineered tag, for example, the peptide or protein, may include, for example, an amino acid motif that may be recognized by isoprenoid transferase. The peptide or protein has deletion at a carboxy terminal of the peptide or protein, or addition by covalent bonds of a spacer unit to the carboxy (C) terminal of the peptide or protein.

The peptide or protein may be directly covalently bonded to the amino acid motif or covalently bonded to the spacer unit to be linked to the amino acid motif. The amino acid spacer unit consists of 1 to 20 amino acids, and among them, a glycine unit is preferred.

The isoprenoid transferase may be, for example, farnesyl protein transferase (FTase) or geranylgeranyl transferase (GGTase), FTase and GGTase I may recognize a CAAX motif in the aforementioned Formula 1, and GGTase II may recognize an XXCC, XCXC, or CXX motif (in which C represents cysteine, A represents an aliphatic amino acid, and X represents an amino acid that determines substrate specificity of isoprenoid transferase).

In yet another embodiment, the linker may include a beta-glucuronide linker that is recognized and hydrolyzed by β-glucuronidase, which is present in large numbers in lysosomes or overexpressed in some tumor cells. Unlike a peptide linker, the beta-glucuronide linker has an advantage of increasing the solubility of the antibody-drug conjugate when linked with a drug having high hydrophobicity due to its high hydrophilicity.

In this regard, the beta-glucuronide linker disclosed in International Patent Publication No. WO2015/182984, for example, a beta-glucuronide linker containing a self-immolative group may be used, and the above document is incorporated by reference.

In some cases, the linker may be, for example, a non-cleavable linker, and the drug is released through only one step of antibody hydrolysis in the cells to produce, for example, an amino acid-linker-drug complex. Such a type of linker may be a thioether group or a maleimidocaproyl group, and may maintain stability in blood.

According to an embodiment of the present invention, the linker-drug may be linked randomly through cysteines exposed when the disulfide bond chain of the antibody is reduced or by introducing an antibody terminal binding peptide having a sequence GGGGGGGCVIM.

The drug (including D of Formula 1) may be an agent that exhibits pharmacological effects and may bind to an antibody, and may specifically be a chemotherapeutic agent, toxin, microRNA (miRNA), siRNA, shRNA, or radioactive isotope. The chemotherapeutic agent may be, for example, a cytotoxic agent or an immunosuppressive agent. Specifically, the chemotherapeutic agent may include a microtubulin inhibitor, a mitotic inhibitor, a topoisomerase inhibitor, or a chemotherapeutic agent that may function as DNA intercalator. In addition, the chemotherapeutic agent may include an immunomodulatory compound, an anticancer agent, an antiviral agent, an antibacterial agent, an antifungal agent, an anthelmintic agent, or a combination thereof.

These drugs may be one or more selected from the group consisting of, for example, maytansinoid, auristatin (including MMAE and MMAF), aminopterin, actinomycin, bleomycin, thalisomycin, camptothecin, N8-acetyl spermidine, 1-(2 chloroethyl)-1,2-dimethyl sulfonyl hydrazide, esperamycin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocamycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, bischloroethylnitrosourea (CNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, 5-ethynyl-1-beta-dribofuranosylimidazole-4-carboxamide (EICAR), hydroxyurea, deferoxamine, fluxuridine, doxifluridine, raltitrexed, cytarabine(ara C), cytosine arabinoside, fludarabine, tamoxifen, raloxifene, megestrol, goserelin, leuprolide acetate, flutamide, bicalutamide, EB 1089, CB1093, KH1060, verteporfin, phthalocyanine, photosensitizer Pe4, demethoxy-hypocrellin A, interferon-α, interferon-γ, tumor necrosis factor, gemcitabine, velcade, revamid, thalamid, lovastatin, 1-methyl-4-phenylpyridiniumion, staurosporine, actinomycin D, dactinomycin, bleomycin A2, bleomycin B2, peplomycin, epirubicin, pirarubicin, zorubicin, mitoxantrone, verapamil, thapsigargin, nuclease, and toxins derived from bacteria, animals, and plants, but are not limited thereto.

In some cases, the drug may include at least one nucleophilic group selected from the group consisting of amine, thiol, hydroxyl, hydrazide, oxime, hydrazine, thiosemicarbazone, hydrazine carboxylate, and arylhydrazide groups which may react to form a covalent bond with a linker and an electrophilic group on the linker reagent.

In a specific embodiment according to the present invention, the antibody or the antigen-binding fragment thereof according to the present invention is linked to a drug, such as auristatin (MMAE), through an MC-vc-PAB linker to prepare an ADC. It was confirmed that such an ADC exhibited the desired cytotoxicity.

### Chimeric Antigen Receptor (CAR)

In another aspect, the present invention relates to a chimeric antigen receptor (CAR) including an extracellular domain including an antigen-binding site, a transmembrane domain, and an intracellular signaling domain, in which the antigen-binding site of the extracellular domain is scFv of the antibody.

The chimeric antigen receptor (CAR) is a synthetic construct designed to induce an immune response against a target antigen and cells expressing the antigen. The CAR includes an extracellular domain, a transmembrane domain, and an intracellular signaling domain. Cancer cells may be killed by introducing a gene encoding a receptor that recognizes a cancer cell surface antigen that is specifically expressed on the surface of cancer cells into immune cells. Through the immune cells containing the receptor that binds to the antigen specifically expressed in the cancer cells, an immune response may be induced by targeting only the cancer cells. The CAR includes the scFv of the anti-PD-1 antibody according to the present invention as an antigen recognition site in the extracellular domain.

A first-generation CAR includes an extracellular domain containing an antigen recognition site specifically expressed in cancer cells, a transmembrane domain, and an intracellular signaling domain, and only CD3ξ was used as a signaling domain, but there was a problem that the therapeutic effect on cancer was insignificant and the duration was short. Such a first-generation CAR is specifically described in US Patent Registration No. 6,319,494, incorporated herein by reference.

A second-generation CAR combining a costimulatory domain (CD28 or CD137/4-1BB) and CD3ξ was prepared to improve the reactivity to immune cells, but compared to the first-generation CAR, the number of CAR-containing immune cells remaining in the body significantly increased. While the second-generation CAR used one costimulatory domain, a third-generation CAR used two or more costimulatory domains. In order to achieve expansion and persistence of immune cells including the CAR *in vivo,* the costimulatory domain may be combined with 4-1BB, CD28, OX40, or the like. The second-generation CAR is specifically described in U.S. Patent No. 7,741,465, 7,446,190, or 9,212,229 and the third-generation CAR is specifically described in U.S. Patent No. 8,822,647, incorporated herein by reference.

A fourth-generation CAR includes an additional gene encoding cytokines such as IL-12 or IL-15, so that CAR-based immunoproteins of cytokines may be further expressed, and a fifth-generation CAR further includes an interleukin receptor chain, for example, IL-2Rβ, to enhance immune cells. The fourth-generation CAR is specifically described in US Patent No. 10,316,102 and the fifth-generation CAR is specifically described in US Patent No. 10,336,810, which are incorporated herein by reference.

In an embodiment, the antigen-binding site of the extracellular domain is scFv of the antibody. In scFv including the VH and VL domains of the antibody, the VH and VL domains may be linked to each other via a linker. A heavy chain variable region including at least one amino acid sequence selected from the group consisting of SEQ ID NOs: 20, 21, 23, 25, 27, 28, 30, 32, 48, and 50 may be linked to a light chain variable region including at least one amino acid sequence selected from the group consisting of SEQ ID NOs: 22, 24, 26, 29, 31, 33, 49, 51, 54, and 55.

The linker may be a peptide linker and have a length of about 10 to 25 aa. For example, hydrophilic amino acids such as glycine and/or serine may be included.

The linker may include, for example, (GS)ₙ, (GGS)ₙ, (GSGGS)ₙ, or (GₙS)ₘ (n and m are each 1 to 10), but the linker may be, for example, (GₙS)ₘ (n and m are each 1 to 10). Specifically, the linker may include GGGGS.

The transmembrane domain may be from natural or synthetic supply sources. In the case of the natural supply sources, the domain may be derived from any membrane-bound protein or transmembrane protein. The transmembrane domain may include a T-cell receptor, CD28, CD3ε, CD45, CD4, CD5, CD8, CD9, CD16, CD22, CD33, CD37, CD64, CD80, CD86, CD134, CD137, CD154, and alpha, beta, or zeta chains of ICOS. When the transmembrane domain is synthesized, hydrophobic residues such as leucine and valine may be included, or peptides including phenylalanine, tryptophan, and valine may be included in each end. A short oligo- or polypeptide linker having a length of 2 and 10 amino acids may form a bond between the transmembrane domain and the cytoplasmic signaling domain of the CAR. A glycine-serine peptide may be used as a linker.

The signaling domain may induce activation of a normal effector function of immune cells in which the CAR is located. For example, cytolytic activation or helper activation may be induced through secretion of cytokines. The signaling domain may include a cleaved fragment of an intracellular signaling domain sufficient to transduce an effector function signal.

The signaling domain may include a cytoplasm of the T cell receptor (TCR) and a co-receptor that act cooperatively to initiate the signaling after participating in the antigen receptor.

It is also known that a signal generated through the TCR alone is insufficient for full activation of T cells and a co-stimulatory signal is required. Accordingly, T cell activation may be involved in initiating antigen-dependent primary activation via the TCR and acting in an antigen-dependent manner to provide secondary or co-stimulatory signals. A primary cytoplasmic signaling sequence regulates primary activation of the TCR complex either in a stimulatory manner or in an inhibitory manner. The primary cytoplasmic signaling sequence that acts in the stimulatory manner may contain a signaling motif known as an immunoreceptor tyrosine-based activation motif or ITAM. Examples of the ITAM containing the primary cytoplasmic signaling sequence may include TCR zeta, FcR gamma, FcR beta, CD3 gamma, CD3 delta, CD3 epsilon, CD5, CD22, CD79a, CD79b, and CD66d.

Optionally, the cytoplasmic domain of the CAR may include a CD3 zeta chain portion and a costimulatory signaling region. The costimulatory signaling region refers to a portion of a CAR that includes an intracellular domain of a costimulatory molecule. For example, the costimulatory signaling region may include ligands and the like, that specifically bind to CD27, CD28, 4-1BB (CD137), OX40, CD30, CD40, ICOS, lymphocyte function-associated antigen-1 (LFA-1), CD2, CD7, LIGHT, NKG2C, B7-H3, and CD83. The cytoplasmic signaling sequence within the cytoplasmic signaling portion of the CAR may be linked via a peptide linker including 2 to 10 amino acids, for example, glycine-serine.

In another aspect, the present invention relates to immune cells into which the chimeric antigen receptor (CAR) is introduced.

The immune cells may induce immunity to induce a desired cancer treatment effect, and may be selected from the group consisting of, for example, T cells, NK cells, cytokine induced killer cells (CIKs), activated cytotoxic T lymphocytes (CTLs), macrophages, tumor-infiltrating lymphocytes (TILs), and dendritic cells, but are not limited thereto.

In some cases, scFv for an antibody other than the anti-PD-1 antibody may be additionally introduced as a chimeric antigen receptor (CAR) included as the antigen-binding site of the extracellular domain.

The antibody other than the anti-PD-1 antibody may be an antibody targeting FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, or NRP1.

### Composition for the treatment

In yet another aspect, the present invention relates to a composition for the treatment of cancer including the antibody or the antigen-binding fragment thereof, the bispecific or multispecific antibody including the antibody or the antigen-binding fragment thereof, the antibody-drug conjugate including the antibody or the antigen-binding fragment thereof, the chimeric antigen receptor including the antibody or the antigen-binding fragment thereof, or the immune cells including the chimeric antigen receptor.

For example, the present invention may be a pharmaceutical composition for preventing or treating cancer including (a) a pharmaceutically effective amount of an antibody or an antigen-binding fragment thereof to PD-1 according to the present invention, a bispecific or multispecific antibody including the antibody or the antigen-binding fragment thereof, an antibody-drug conjugate including the antibody or the antigen-binding fragment thereof, a chimeric antigen receptor including the antibody or the antigen-binding fragment thereof, or immune cells including the chimeric antigen receptor; and (b) a pharmaceutically acceptable carrier. Further, the present invention may be a method for preventing or treating cancer including administering, to cancer patients, an antibody or an antigen-binding fragment thereof to PD-1 according to the present invention, a bispecific or multispecific antibody including the antibody or the antigen-binding fragment thereof, an antibody-drug conjugate including the antibody or the antigen-binding fragment thereof, a chimeric antigen receptor including the antibody or the antigen-binding fragment thereof, or immune cells including the chimeric antigen receptor.

The "prevention" refers to any action that suppresses clinical symptoms or delays the progression of a disease by administering the composition according to the present invention, and the "treatment" refers to inhibition of development of clinical symptoms of the disease, and alleviation or elimination of clinical symptoms of the disease.

The cancer includes, for example, Hodgkin's lymphoma, non-Hodgkin's lymphoma (e.g., B cell lymphoma, diffuse large B cell lymphoma, follicular lymphoma, chronic lymphocytic leukemia, mantle cell lymphoma, marginal zone B cell lymphoma, Burkitt's lymphoma, lymphoplasmacytic lymphoma, hairy cell leukemia), acute myelogenous leukemia, chronic myelogenous leukemia, myelodysplastic syndrome, multiple myeloma, or acute lymphocytic leukemia.

The cancer includes, for example, ovarian cancer, rectal cancer, stomach cancer, testicular cancer, anal region cancer, uterine cancer, colon cancer, rectal cancer, renal cell carcinoma, liver cancer, non-small cell carcinoma of the lung, small intestine cancer, esophageal cancer, melanoma, Kaposi's sarcoma, endocrine cancer, thyroid cancer, parathyroid cancer, adrenal cancer, bone cancer, pancreatic cancer, skin cancer, head and neck cancer, skin or intraocular malignant melanoma, uterine cancer, brain stem glioma, pituitary adenocarcinoma, epidermal cancer, carcinoma of cervical squamous cell cancer, fallopian tube carcinoma, endometrial carcinoma, vaginal carcinoma, soft tissue sarcoma, urethral cancer, vulvar carcinoma, penile cancer, bladder cancer, kidney or ureter cancer, renal pelvic carcinoma, spinal tumor, neoplasia of the central nervous system (CNS), primary CNS lymphoma, tumor angiogenesis, metastatic lesions of the cancers, or combinations of the cancers.

The cancer may be, for example, glioblastoma, lung cancer, bladder cancer, oral cancer, head and neck squamous cell cancer, gallbladder cancer, or cervical cancer. In particular, in the case of the glioblastoma, it is required to pass through a blood-brain barrier, which is a barrier formed by a tight junction in a capillary endothelial cell membrane of the brain, where the antibody or the antigen-binding fragment thereof is present between the brain and spine and the surrounding circulatory system. The blood-brain barrier may be used in connection with a carrier to pass through the BBB. To deliver the drug through the BBB, there is a method of disrupting the osmotic pressure of the BBB by using methods such as bradykinin or high density foucues ultrasound (HIFU). In addition, the method may also include the use of delivery systems such as glucose and amino acid carriers embedded in cells and receptor-mediated transcytosis of insulin or transferrin, or blocking an active efflux transporter of glycoproteins.

The pharmaceutically acceptable carrier to be included in the composition of the present invention is generally used in preparation, and includes lactose, dextrose, sucrose, sorbitol, mannitol, starch, acacia rubber, calcium phosphate, alginate, gelatin, calcium silicate, microcrystalline cellulose, polyvinylpyrrolidone, water, syrup, methylcellulose, methylhydroxybenzoate, propylhydroxybenzoate, talc, magnesium stearate, mineral oil, and the like, but is not limited thereto. The composition of the present invention may further include a lubricant, a wetting agent, a sweetening agent, a flavoring agent, an emulsifying agent, a suspending agent, a preservative, and the like, in addition to the ingredients.

The pharmaceutical composition of the present invention may be administered orally or parenterally, and in the case of parenteral administration, the pharmaceutical composition may be administered through intravenous injection, subcutaneous injection, intramuscular injection, intraperitoneal injection, intradermal administration, topical administration, intranasal administration, intrapulmonary administration, intrarectal administration, etc.

In oral administration, since the protein or peptide is digested, it is preferable to formulate the oral composition so as to coat an active agent or to be protected from decomposition in the stomach. In addition, the pharmaceutical composition may be administered by any device capable of moving the active material to a target cell.

A suitable dose of the composition according to the present invention varies depending on factors, such as a formulation method, an administration method, and age, weight, sex, medical condition, food, an administration time, an administration route, an excretion rate, and response sensitivity of a patient, and an ordinarily skilled physician may easily determine and prescribe a dose effective for desired treatment or prevention. For example, a daily dose of the pharmaceutical composition of the present invention is 0.0001 to 100 mg/kg. As used herein, the term "pharmaceutically effective amount" means an amount sufficient to prevent or treat cancer.

The pharmaceutical composition of the present invention may be formulated by using a pharmaceutically acceptable carrier and/or excipient according to a method that may be easily performed by those skilled in the art to be prepared in a unit dose form or prepared by introduction into a multi-dose container. In this case, the formulation may also be forms of solutions, suspensions, or emulsions in oils or aqueous media or forms of extracts, powders, suppositories, powders, granules, tablets, or capsules, and may additionally include a dispersant or a stabilizer.

### Combination therapy

The present invention relates to a composition for combination therapy including immune cells and a drug other than an anti-PD-1 antibody.

In one embodiment, the drug other than the anti-PD-1 antibody may include a chemotherapeutic agent or an antibody other than the anti-PD-1 antibody.

The drug may be at least one selected from the group consisting of maytansinoid, auristatin (including MMAE and MMAF), aminopterin, actinomycin, bleomycin, thalisomycin, camptothecin, N8-acetyl spermidine, 1-(2 chloroethyl)-1,2-dimethyl sulfonyl hydrazide, esperamycin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocamycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, bischloroethylnitrosourea (CNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, 5-ethynyl-1-beta-dribofuranosylimidazole-4-carboxamide (EICAR), hydroxyurea, deferoxamine, fluxuridine, doxifluridine, raltitrexed, cytarabine(ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-α, interferon-γ, tumor necrosis factor, gemcitabine, velcade, revamid, thalamid, lovastatin, 1-methyl-4-phenylpyridiniumion, staurosporine, actinomycin D, dactinomycin, bleomycin A2, bleomycin B2, peplomycin, epirubicin, pirarubicin, zorubicin, mitoxantrone, verapamil, thapsigargin, nuclease, and toxins derived from bacteria, animals, and plants.

In one embodiment, the antibody other than the anti-PD-1 antibody may be an antibody or an antigen-binding fragment thereof targeting at least one selected from the group consisting of, for example, FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

The present invention relates to a composition for combination therapy including an antibody or an antigen-binding fragment thereof and at least one selected from the group consisting of the following:
(i) immune cells;
(ii) immune cells comprising an antigen receptor (CAR) containing scFv for an antibody other than an anti-PD-1 antibody as an extracellular domain; and
(iii) an immune checkpoint inhibitor.

Further, the present invention relates to a composition for combination therapy including the immune cell engaging bispecific or multispecific antibody and at least one selected from the group consisting of the following:
(i) immune cells;
(ii) immune cells comprising an antigen receptor (CAR) containing an scFv fragment for an antibody other than an anti-PD-1 antibody as an extracellular domain; and
(iii) an immune checkpoint inhibitor.

The immune cells may induce a desired cancer treatment effect by inducing immune therapy, for example, immunity, and may be selected from the group consisting of, for example, T cells, NK cells, cytokine induced killer cells (CIKs), activated cytotoxic T lymphocytes (CTLs), macrophages, tumor-infiltrating lymphocytes (TILs), and dendritic cells, but are not limited thereto.

The antibody other than the anti-PD-1 antibody may be an antibody targeting a target other than PD-1, and may be an antibody or an antigen-binding fragment binding to, for example, FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, or NRP1, but is not limited thereto.

The immune checkpoint inhibitor refers to an agent capable of inducing T cell activation by blocking T cell inhibitory signals to a site where antigen presenting cells (APCs) and immune cells, for example, T cells meet. The immune checkpoint inhibitor may be a drug targeting, for example, TIGIT, PD-L1, BTLA, CTLA-4, VISTA, LAG3, TIM3, CD137(4-1BB), CD258(LIGHT), MARCO, CD134(OX40), CD28, CD278(ICOS), CD27, CD154(CD40L), CD357(GITR), CD30, DR3, CD226(DNAM1), CD96, CD200, or CD200R, but is not limited thereto.

A first ingredient and a second ingredient to be administered in combination may be administered simultaneously. In addition, the first ingredient and the second ingredient to be administered in combination may also be separately administered at a predetermined time interval. The second ingredient may be separately administered before or after administration of the first ingredient to be administered in combination.

### Companion diagnostics (CDx)

Companion diagnostics is a type of molecular diagnostic technique for predicting a patient's responsiveness to a specific drug treatment, and may present a screening reference of patients to be applied in the application of drugs and present a reference for treating a drug suitable for the patients in consideration of the specificity and sensitivity of diagnostic, prognostic, and predictive biomarkers that correspond thereto.

Since the anticancer drug is highly effective in patients showing a response, but has a low ratio of patients showing a response, it is important to select patients who have the effect, and through companion diagnosis, it is possible to select patients who respond to the anticancer drug and increase the treatment efficiency of patients.

The sample refers to a biological subject obtained from a patient's tissue or bodily fluid. The source of the tissue sample may be solid tissue derived from a frozen and/or preserved organ, tissue sample, biopsy, or aspirate; blood or any blood component (e.g., serum, plasma); bone marrow or any bone marrow component; and a bodily fluid such as urine, cerebrospinal fluid, whole blood, plasma, and serum. The sample may include a non-cellular fraction (e.g., urine, plasma, serum, or other non-cellular bodily fluid). The sample may include a bodily fluid, such as blood (e.g., whole blood). Specifically, the sample may be a whole blood sample, a whole bone marrow sample, a whole peripheral blood sample, or a whole tumor sample obtained from patients. The "whole" sample is a sample that is substantially free of components (e.g., cells) that have been removed or isolated from the sample. The sample, for example, a blood sample may be diluted (e.g., by a physiologically compatible buffer or medium) before use. In other embodiments, the "whole" sample, such as a whole tissue sample or a whole tumor sample, may substantially maintain an origin tissue-derived microenvironment and substantially maintain a structure of, for example, a tumor or immune microenvironment. Specifically, the sample, for example, the tumor sample may be processed (e.g., ground, minced, blended, pulverized, etc.) into smaller pieces and diluted (e.g., by a physiologically compatible buffer or medium).

The confirmation of the expression of a gene is a process of confirming the presence and the expression level of the corresponding gene, and may be performed by polymerase chain reaction (PCR). In some cases, the confirmation may be performed by using RT-PCR, competitive reverse transcription polymerase chain reaction (competitive RT-PCR), real-time RT-PCR, RNase protection assay (RPA), Northern blotting, and DNA chips.

Polymerase chain reaction (PCR) may be performed through a gene amplification reaction using primers. Multiplex PCR, real-time PCR, differential display PCR (DD-PCR), rapid amplification of cDNA ends (RACE), inverse polymerase chain reaction (IPCR), vectorette PCR, thermal asymmetric interlaced PCR (TAII,-PCR), and the like may be performed.

It is also possible to detect whether a protein is expressed by genetic coding. This is a process of confirming the presence and the expression level of a protein expressed from the gene. The amount of the protein may be confirmed using an antibody, an oligopeptide, a ligand, a peptide nucleic acid (PNA), or an aptamer that binds specifically to the protein.

As the analysis method for this, there may be included Western blotting, enzyme linked immunosorbent assay (ELISA), radioimmunoassay (RIA), radioimmunodiffusion, Ouchterlony immunodiffusion, rocket immunoelectrophoresis, tissue immunostaining, immunoprecipitation assay, complement fixation assay, fluorescence activated cell sorter (FACS), a method using protein chips, etc.

### Example

Hereinafter, the present invention will be described in more detail through Examples. These Examples are just illustrative of the present invention, and it will be apparent to those skilled in the art that it is not interpreted that the scope of the present invention is limited to these Examples.

### Example 1. Construction of candidate clones and analysis of antigen binding affinity

As illustrated in FIG. 1, a full-length PD-1 DNA sequence was cloned into a vaccination vector and used as a DNA vaccination, and combined with a recombinant human PD-1-His protein to immunize mice and also prepare a 293 cell line stably expressing human PD-1. Blood was collected before and after immunization to confirm the degree of immunization. After secondary DNA vaccination, the serum was isolated and bound to the 293 cell line expressing human PD-1 and analyzed, and as a result, it was confirmed that an anti-PD-1 antibody was produced in the serum of mice Nos. 1, 2, and 4. After immunization with a tertiary protein, the serum was isolated and bound to the 293 cell line expressing human PD-1 and analyzed, and as a result, it was confirmed that an anti-PD-1 antibody was produced in the serum of all 5 mice. Thereafter, DNA vaccination and protein immunization were administered once more, and hybridoma clones were generated by fusion of the spleen of mice Nos. 2 and 4 with SP2/0 myeloid cells.

As illustrated in FIG. 2, primary 11 clones were selected from clones having binding affinity to a recombinant human PD-1-His protein using a hybridoma culture medium by an ELISA method.

As illustrated in FIG. 3, after 1 × 10⁶ cells of each clone of 11 hybridomas were cultured for 24 hours, ELISA was performed using the culture medium to quantitatively compare the binding affinity of the antibody. For ELISA, the recombinant human PD-1-His protein was O/N coated on a 96-well ELISA plate at 4°C, and blocked with 5% non-fat skim milk at RT for 1 hr. The antibody was treated with 1/2 serial dilution of each hybridoma culture medium from 100 ul at RT for 2 hr, and detected by anti-mIgG-HRP. As a result, 9 secondary candidate antibodies of 2-2B3, 2-3C3, 2-5F1, 2-7C5, 2-9A9, 4-8D4, 4-8H11, 4-9D12, and 4-9F10 were selected from the selected primary candidate antibodies.

### Example 2. Characteristics of selected clones

As illustrated in FIG. 4, 9 candidate antibodies selected from the hybridoma culture medium were purified and the characteristics thereof were analyzed by SDS-PAGE (4A), SEC-HPLC (4B), and SPR (4C). The 9 clones showed similar binding affinity to the binding affinity with the recombinant human PD-1-His protein, and among the clones, 4-9D12 and 4-9F10 clones showed relatively higher binding affinity.

As illustrated in FIGS. 5 and 6, as a result of analyzing the binding affinity of the candidate antibodies purified using some of PD-1 expressing effector cells used in the PD-1/PD-L1 blocking assay by flow cytometry, 2-2B3 (49.9%), 2-7C5 (50.4%), 4-8D4 (23.5%), and the like were confirmed.

As illustrated in FIG. 6, when effector cells expressing PD-1 and aAPC cells expressing PD-L1, which are two genetically engineered cell lines, were co-cultured for *in vitro* efficacy analysis of the purified candidate antibodies, the binding of PD-1 and PD-L1 interfered with a TCR signal to suppresses luminescence. However, when the binding of PD-1 and PD-L1 was blocked by the efficacy of the selected candidate antibodies, the luminescence was induced by activation of the TCR signal. Therefore, the above-described principle was applied. The selected antibodies were 2-fold serial diluted from 5 µg, and as a result of confirming the blocking effect on the binding of PD-1 and PD-L1, the selected antibodies exhibited a similar inhibitory effect to a positive control anti-PD-1 antibody (Promega) in 2-2B3, 2-7C5, and 4-8D4 clones.

Accordingly, three candidate antibodies were selected based on the above results, and RNA was isolated from each hybridoma and cDNA was synthesized from the selected antibodies for cDNA cloning. The antibody fragments of each antibody, VH and VL, were amplified by RACE PCR, and the amplified PCR product was cloned into a pGEM-Teasy vector, and then the sequence was analyzed. As a result, the selected 3 clones were antibodies having different base and amino acid sequences, respectively, and these 3 antibodies were humanized.

### Example 3, Sequencing of selected individual clones

The results of VH, VL, and CDR region sequence analysis of the selected clones were summarized in Table 1. Among the selected clones, two clones of 2-2B3 were represented as 2-2B3W and 2-2B3R.

2-2B3W included a heavy chain variable region VH of SEQ ID NO: 20 and a light chain variable region VL of SEQ ID NO: 22; VH CDR1 was GFVFSNYD (SEQ ID NO: 1), VH CDR2 was ITIGGGTT (SEQ ID NO: 2), VH CDR3 was ARWVYDPLYAMDY (SEQ ID NO: 3), VL CDR1 was ESVDNYGISF (SEQ ID NO: 5), VL CDR2 was AAS (SEQ ID NO: 6), and VL CDR3 was QQSKEVPWT (SEQ ID NO: 7);

2-2B3R included a heavy chain variable region VH of SEQ ID NO: 21 and a light chain variable region VL of SEQ ID NO: 22; VH CDR1 was GFVFSNYD (SEQ ID NO: 1), VH CDR2 was ITIGGGTT (SEQ ID NO: 2), VH CDR3 was ARRVYDPLYAMDY (SEQ ID NO: 4), VL CDR1 was ESVDNYGISF (SEQ ID NO: 5), VL CDR2 was AAS (SEQ ID NO: 6), and VL CDR3 was QQSKEVPWT (SEQ ID NO: 7);

2-7C5 included a heavy chain variable region VH of SEQ ID NO: 23 and a light chain variable region VL of SEQ ID NO: 24; VH CDR1 was GFTFSSFG (SEQ ID NO: 8), VH CDR2 was IYSNGDYT (SEQ ID NO: 9), VH CDR3 was ARYYGNYGGYFDY (SEQ ID NO: 10), VL CDR1 was QSVSNDVA (SEQ ID NO: 11), VL CDR2 was YAS (SEQ ID NO: 12), and VL CDR3 was QQDYSSPPT (SEQ ID NO: 13); and

4-8D4 included a heavy chain variable region VH of SEQ ID NO: 25 and a light chain variable region VL of SEQ ID NO: 26; VH CDR1 was GYTFTTYW (SEQ ID NO: 14), VH CDR2 was INPTTGYT (SEQ ID NO: 15), VH CDR3 was ARGVRYYFDY (SEQ ID NO: 16), VL CDR1 was QSLLDSRTRKNY (SEQ ID NO: 17), VL CDR2 was WAS (SEQ ID NO: 18), and VL CDR3 was KQSYNLIT (SEQ ID NO: 19).

**[Table 1]**

| Antibody sequence | | | |
|---|---|---|---|
| **Clone** | **Classification** | **Amino acid sequence** | **SEQ ID NO:** |
| **2-2B3W** | **VH** | MDSRLNLVFLVLTLKGVKCEVQLVESGGGLVKPG GSLKLSCAASGFVFSNYDMSWVRQTPEKRLEW VAYITIGGGTTYYPDTVKGRFTISRDNAKNTLY LQMNSLKSEDTAIYYCARWVYDPLYAMDYWG QGTSVTVSS | 20 |
| | **VL** | METDTLLLWVLLLWVPGSTGDILLTQSPASLAVSL GQRATISCRASESVDNYGISFMNWFQQKPGQPP KLLIYAASNQGSGVPARFSGSGSGTDFSLNIHPM EEDDTAMYFCQQSKEVPWTFGGGTKLEIK | 22 |
| **2-2B3R** | **VH** | MDSRLNLVFLVLTLKGVKCEVQLVESGGGLVKPG GSLKLSCAASGFVFSNYDMSWVRQTPEKRLEW VAYITIGGGTTYYPDTVKGRFTISRDNAKNTLY LQMNSLKSEDTAIYYCARRVYDPLYAMDYWG QGTSVTVSS | 21 |
| | **VL** | METDTLLLWVLLLWVPGSTGDILLTQSPASLAVSL GQRATISCRASESVDNYGISFMNWFQQKPGQPP KLLIYAASNQGSGVPARFSGSGSGTDFSLNIHPM EEDDTAMYFCQQSKEVPWTFGGGTKLEIK | 22 |
| **2-7C5** | **VH** | MDSRLNLVFLALILKGVQCEVQLVESGGGLVQPV GSLKLSCAASGFTFSSFGMSWVRQTPDKRLEL VATIYSNGDYTYYPDSVKGRFTVSRDNAKNTL YLQMSSLKSEDTAMYYCARYYGNYGGYFDY WGQGTTLTVSS | 23 |
| | **VL** | MESQTQVFVFLLLCVSGAHGSIVMTQTPKFLLVS AGDRVTITCKASQSVSNDVAWYQQKPGQSPKL LIYYASNRYTGAPDRFTGSGYGTDFTFTISTVQA EDLAVYFCQQDYSSPPTFGGGTKLEIK | 24 |
| **4-8D4** | **VH** | MECTWVILFLFSVTAGVHSQVQLQQSGAELAKPG ASVKMSCKASGYTFTTYWIHWVKQRPGQGLE WIGYINPTTGYTEYNQKFKDKATLTADKSSNT AYMQLSSLTSEDSAVYYCARGVRYYFDYWGK GTTLTVSS | 25 |
| | **VL** | MGFKMESQSLVLILLLLWVSGSCGDIVMSQSPSSL AVSAGEKVTMSCKSSQSLLDSRTRKNYLAWY QQKPGQSPKLLIYWASTRESGVPDRFTGSGSGT | 26 |
| | | DFTLTISSVKAEDLAVYLCKQSYNLITFGAGTK LELK | |

Signal peptides was shown in italics. A VH CDR region and a VL CDR region of each clone were shown in bold.

**[Table 2]**

| | | |
|---|---|---|
| Nucleic acid sequence | | |

| | **Optimized Nucleotide sequence** | **SEQ ID NO:** |
|---|---|---|
| **2-2B3W-VH** | ATGGACTCCAGGCTCAATTTAGTTTTCCTTGTCCTTACTTTA AAAGGTGTGAAGTGTGAAGTGCAGCTGGTGGAGTCTGGGG GAGGCTTAGTGAAGCCTGGAGGGTCCCTGAAACTCTCCTGT GCAGCCTCTGGATTCGTTTTCAGTAACTATGACATGTCTTGG GTTCGCCAGACTCCGGAGAAGAGGCTGGAGTGGGTCGCAT ACATTACTATTGGTGGTGGTACCACCTACTATCCAGACACT GTGAAGGGCCGATTCACCATCTCCAGAGACAATGCCAAGA ACACCCTGTACCTTCAAATGAACAGTCTGAAGTCTGAGGAC ACAGCCATATATTACTGTGCAAGGTGGGTCTATGATCCTCT CTATGCTATGGACTACTGGGGTCAAGGAACCTCAGTCACCG TCTCCTCA | 34 |
| **2-2B3W-VL** | ATGGAGACAGACACACTCCTGCTATGGGTCCTGCTTCTCTG GGTTCCAGGTTCCACAGGTGACATTTTGCTGACCCAATCTCC AGCTTCTTTGGCTGTGTCTCTAGGGCAGAGGGCCACCATCT CCTGCAGAGCCAGCGAAAGTGTTGATAATTATGGCATTAGT TTTATGAACTGGTTCCAACAGAAACCAGGACAGCCACCCAA ACTCCTCATCTATGCTGCATCCAACCAAGGATCCGGGGTCC CTGCCAGGTTTAGTGGCAGTGGGTCTGGGACAGACTTCAGC CTCAACATCCATCCTATGGAGGAGGATGATACTGCAATGTA TTTCTGTCAGCAAAGTAAGGAGGTTCCGTGGACGTTCGGTG GAGGCACCAAGCTGGAAATCAAA | 36 |
| **2-2B3R-VH** | ATGGACTCCAGGCTCAATTTAGTTTTCCTTGTCCTTACTTTA AAAGGTGTGAAGTGTGAAGTGCAGCTGGTGGAGTCTGGGG GAGGCTTAGTGAAGCCTGGAGGGTCCCTGAAACTCTCCTGT GCAGCCTCTGGATTCGTTTTCAGTAACTATGACATGTCTTGG GTTCGCCAGACTCCGGAGAAGAGGCTGGAGTGGGTCGCAT ACATTACTATTGGTGGTGGTACCACCTACTATCCAGACACT GTGAAGGGCCGATTCACCATCTCCAGAGACAATGCCAAGA | 35 |
| | ACACCCTGTACCTTCAAATGAACAGTCTGAAGTCTGAGGAC ACAGCCATATATTACTGTGCAAGGCGGGTCTATGATCCTCT CTATGCTATGGACTACTGGGGTCAAGGAACCTCAGTCACCG TCTCCTCA | |
| **2-2B3R-VL** | ATGGAGACAGACACACTCCTGCTATGGGTCCTGCTTCTCTG GGTTCCAGGTTCCACAGGTGACATTTTGCTGACCCAATCTCC AGCTTCTTTGGCTGTGTCTCTAGGGCAGAGGGCCACCATCT CCTGCAGAGCCAGCGAAAGTGTTGATAATTATGGCATTAGT TTTATGAACTGGTTCCAACAGAAACCAGGACAGCCACCCAA ACTCCTCATCTATGCTGCATCCAACCAAGGATCCGGGGTCC CTGCCAGGTTTAGTGGCAGTGGGTCTGGGACAGACTTCAGC CTCAACATCCATCCTATGGAGGAGGATGATACTGCAATGTA TTTCTGTCAGCAAAGTAAGGAGGTTCCGTGGACGTTCGGTG GAGGCACCAAGCTGGAAATCAAA | 36 |
| **2-7C5-VH** | ATGGACTCCAGGCTCAATTTAGTTTTCCTTGCCCTTATTTTA AAAGGTGTCCAGTGTGAGGTGCAGCTGGTGGAGTCTGGGG GAGGCTTAGTGCAGCCTGTAGGGTCCCTGAAACTCTCCTGT GCAGCCTCTGGATTCACTTTCAGTAGTTTTGGCATGTCTTGG GTTCGCCAGACTCCAGACAAGAGGCTGGAGTTGGTCGCAAC CATTTATAGTAATGGTGATTACACCTATTATCCAGACAGTGT GAAGGGCCGATTCACCGTCTCCAGAGACAATGCCAAGAAC ACCCTGTACCTGCAAATGAGCAGTCTGAAGTCTGAGGACAC AGCCATGTATTACTGTGCAAGGTACTATGGTAACTACGGGG GGTACTTTGACTACTGGGGCCAAGGCACCACTCTCACAGTC TCCTCA | 37 |
| **2-7C5-VL** | ATGGAGTCACAGACCCAGGTCTTTGTATTTCTACTGCTCTGT GTGTCTGGTGCTCATGGGAGTATTGTGATGACCCAGACTCC CAAATTCCTGCTTGTATCAGCAGGAGACAGGGTTACCATAA CCTGCAAGGCCAGTCAGAGTGTGAGTAATGATGTAGCTTGG TACCAACAGAAGCCAGGGCAGTCTCCTAAATTGCTGATATA CTATGCATCCAATCGCTACACTGGAGCCCCTGATCGCTTCA CTGGCAGTGGATATGGGACGGATTTCACTTTCACCATCAGC ACTGTGCAGGCTGAAGACCTGGCAGTTTATTTCTGTCAGCA GGATTATAGCTCTCCTCCGACATTCGGTGGAGGCACCAAGC TGGAAATCAAA | 38 |
| **4-8D4-VH** | ATGGAATGCACCTGGGTCATTCTCTTCCTGTTTTCAGTAACT GCAGGTGTCCACTCCCAGGTCCAGCTTCAGCAGTCTGGGGC | 39 |
| | TGAACTGGCAAAACCTGGGGCCTCAGTGAAGATGTCCTGCA AGGCTTCTGGCTACACCTTTACTACCTACTGGATTCACTGGG TAAAACAGAGGCCTGGACAGGGACTGGAATGGATTGGATA CATTAATCCTACCACTGGTTATACTGAATACAATCAGAAGT TCAAGGACAAGGCCACATTGACTGCAGACAAATCCTCCAAC ACAGCCTACATGCAACTGAGCAGCCTGACATCTGAGGACTC TGCAGTCTATTACTGTGCAAGAGGAGTACGGTACTACTTTG ACTACTGGGGCAAAGGCACCACTCTCACAGTCTCCTCA | |
| 4-8D4-VL | ATGGGCTTCAAGATGGAGTCACAGTCTCTGGTTCTTATATTG CTGCTGCTATGGGTATCTGGTTCCTGTGGGGACATTGTGATG TCACAGTCTCCATCCTCCCTGGCTGTGTCAGCAGGAGAGAA GGTCACTATGAGCTGCAAATCCAGTCAGAGTCTGCTCGACA GTAGAACCCGAAAGAACTACTTGGCTTGGTACCAGCAGAA ACCAGGGCAGTCTCCTAAACTGCTGATCTACTGGGCATCCA CTAGGGAATCTGGGGTCCCTGATCGCTTCACAGGCAGTGGA TCTGGGACAGATTTCACTCTCACCATCAGCAGTGTGAAGGC TGAAGACCTGGCAGTTTATCTCTGCAAGCAATCTTATAATCT GATCACGTTCGGTGCTGGGACCAAGCTGGAGCTGAAA | 40 |

### Example 4. Preparation of humanized antibodies

From the results of Examples 1 to 3, humanized antibodies 2B3W, 2B3R, 7C5, and 8D4 against clones 2-2B3W, 2-2B3R, 2-7C5, and 4-8D4 were prepared. The results of analyzing the sequences of the prepared humanized antibodies were shown in Table 3.
2B3W included a heavy chain variable region VH of SEQ ID NO: 27 and a light chain variable region VL of SEQ ID NO: 29; VH CDR1 was GFVFSNYD (SEQ ID NO: 1), VH CDR2 was ITIGGGTT (SEQ ID NO: 2), VH CDR3 was ARWVYDPLYAMDY (SEQ ID NO: 3), VL CDR1 was ESVDNYGISF (SEQ ID NO: 5), VL CDR2 was AAS (SEQ ID NO: 6), and VL CDR3 was QQSKEVPWT (SEQ ID NO: 7);
2B3R included a heavy chain variable region VH of SEQ ID NO: 28 and a light chain variable region VL of SEQ ID NO: 29; VH CDR1 was GFVFSNYD (SEQ ID NO: 1), VH CDR2 was ITIGGGTT (SEQ ID NO: 2), VH CDR3 was ARRVYDPLYAMDY (SEQ ID NO: 4), VL CDR1 was ESVDNYGISF (SEQ ID NO: 5), VL CDR2 was AAS (SEQ ID NO: 6), and VL CDR3 was QQSKEVPWT (SEQ ID NO: 7);
7C5 included a heavy chain variable region VH of SEQ ID NO: 30 and a light chain variable region VL of SEQ ID NO: 31; VH CDR1 was GFTFSSFG (SEQ ID NO: 8), VH CDR2 was IYSNGDYT (SEQ ID NO: 9), VH CDR3 was ARYYGNYGGYFDY (SEQ ID NO: 10), VL CDR1 was QSVSNDVA (SEQ ID NO: 11), VL CDR2 was YAS (SEQ ID NO: 12), and VL CDR3 was QQDYSSPPT (SEQ ID NO: 13); and
8D4 included a heavy chain variable region VH of SEQ ID NO: 32 and a light chain variable region VL of SEQ ID NO: 33; VH CDR1 was GYTFTTYW (SEQ ID NO: 14), VH CDR2 was INPTTGYT (SEQ ID NO: 15), VH CDR3 was ARGVRYYFDY (SEQ ID NO: 16), VL CDR1 was QSLLDSRTRKNY (SEQ ID NO: 17), VL CDR2 was WAS (SEQ ID NO: 18), and VL CDR3 was KQSYNLIT (SEQ ID NO: 19).

**[Table 3]**

| | Humanized antibody sequence | | |
|---|---|---|---|
| **Clone** | **Classification** | **Amino acid sequence** | **SEQ ID NO:** |
| **2B3W** | **VH** | EVQLVESGGGLVQPGGSLRLSCAASGFVFSNYDMS WVRQAPGKGLEWVAYITIGGGTTYYPDTVKGRFT ISRDNAKNSLYLQMNSLRAEDTAVYYCARWVYDP LYAMDYWGQGTTVTVSS | 27 |
| | **VL** | DIVLTQTPLSLSVTPGQPASISCRASESVDNYGISFM NWFLQKPGQPPQLLIYAASNQGSGVPDRFSGSGSG | 29 |
| | | TDFTLKISRVEAEDVGVYYCQQSKEVPWTFGQGT KVEIK | |
| **2B3R** | **VH** | EVQLVESGGGLVQPGGSLRLSCAASGFVFSNYDMS WVRQAPGKGLEWVAYITIGGGTTYYPDTVKGRFT ISRDNAKNSLYLQMNSLRAEDTAVYYCARRVYDP LYAMDYWGQGTTVTVSS | 28 |
| | **VL** | DIVLTQTPLSLSVTPGQPASISCRASESVDNYGISFM NWFLQKPGQPPQLLIYAASNQGSGVPDRFSGSGSG TDFTLKISRVEAEDVGVYYCQQSKEVPWTFGQGT KVEIK | 29 |
| **2B3W-T14-05** | **VH** | EVQLVESGGGLVQPGGSLRLSCAASGFVFSNYDMS WVRQAPGKGLEWVAYITIGGGTTYYPDTVKGRFT ISRDNAKNSLYLQMNSLRAEDTAVYYCARWRYDP LFAMDYWGQGTTVTVSS | 48 |
| | **VL** | DIVL TQTPLSLSVTPGQPASISCRASESVTDYGISFM NWFLQKPGQPPQLLIYAASNQGSGVPDRFSGSGSG TDFTLKISRVEAEDVGVYYCQQSKEVPWTFGQGT KVEIK | 49 |
| **7C5** | **VH** | EVQLVESGGGLVKPGGSLRLSCAASGFTFSSFGMS WVRQAPGKGLELVATIYSNGDYTYYPDSVKGRFT VSRDNAKNSLYLQMNSLRAEDTAVYYCARYYGN YGGYFDYWGQGTLVTVSS | 30 |
| | **VL** | AIQMTQSPSSLSASVGDRVTITCKASQSVSNDVAW YQQKPGKAPKLLIYYASNRYTGVPSRFSGSGSGTD FTLTISSLQPEDFATYYCQQDYSSPPTFGGGTKVEI K | 31 |
| **7C5-T15-01** | **VH** | EVQLVESGGGLVKPGGSLRLSCAASGFTFSSFGMS WVRQAPGKGLELVATIYSNGDYTYYPDSVKGRFT VSRDNAKNSLYLQMNSLRAEDTAVYYCARYYGN YGGYFDYWGQGTLVTVSS | 50 |
| | **VL** | AIQMTQSPSSLSASVGDRVTITCKASQSVWDDLTW YQQKPGKAPKLLIYYASNRYTGVPSRFSGSGSGTD FTLTISSLQPEDFATYYCQQDYSSPPTFGGGTKVEI K | 51 |
| **8D4** | **VH** | QVQLVQSGAEVKKPGASVKVSCKASGYTFTTYWI HWVRQAPGQGLEWIGYINPTTGYTEYNQKFKDRA | 32 |
| | | TLTADKSTSTAYMELSSLRSEDTAVYYCARGVRY YFDYWGKGTTVTVSS | |
| | **VL** | DIVMTQSPDSLAVSLGERATINCKSSQSLLDSRTRK NYLAWYQQKPGQPPKLLIYWASTRESGVPDRFSGS GSGTDFTLTISSLQAEDVAVYYCKQSYNLITFGQG TKLEIK | 33 |
| **8D4-T16-01** | **VH** | QVQLVQSGAEVKKPGASVKVSCKASGYTFTTYWI HWVRQAPGQGLEWIGYINPTTGYTEYNQKFKDRA TLTADKSTSTAYMELSSLRSEDTAVYYCARGVRY YFDYWGKGTTVTVSS | 52 |
| | **VL** | DIVMTQSPDSLAVSLGERATINCKSSQSLLDSRTRK NYLAWYQQKPGQPPKLLIYWASTRESGVPDRFSGS GSGTDFTLTISSLQAEDVAVYYCKQSYNLITFGQG TKLEIK | 53 |
| **8D4-T 16-02** | **VH** | QVQLVQSGAEVKKPGASVKVSCKASGYTFTTYWI HWVRQAPGQGLEWIGYINPTTGYTEYNQKFKDRA TLTADKSTSTAYMELSSLRSEDTAVYYCARGVRY YFDYWGKGTTVTVSS | 54 |
| | **VL** | DIVMTQSPDSLAVSLGERATINCKSSQSLLDSRTRK NYLAWYQQKPGQPPKLLIYWASTRESGVPDRFSGS GSGTDFTLTISSLQAEDVAVYYCQQQYDPPITFGQ GTKLEIK | 55 |

A VH CDR region and a VL CDR region of each clone were shown in bold. Engineered amino acid residues of each clone were underlined.

**[Table 4]**

| | Nucleic acid sequence | |
|---|---|---|
| | **Optimized Nucleotide sequence** | **SEQ ID NO:** |
| **2B3W-VH** | GAGGTGCAGCTGGTGGAGAGCGGCGGCGGCCTGGTGCAG CCCGGCGGCAGCCTGAGGCTGAGCTGCGCCGCCAGCGGCT TCGTGTTCAGCAACTACGACATGAGCTGGGTGAGGCAGGC CCCCGGCAAGGGCCTGGAGTGGGTGGCCTACATCACCATC GGCGGCGGCACCACCTACTACCCCGACACCGTGAAGGGC AGGTTCACCATCAGCAGGGACAACGCCAAGAACAGCCTG | 41 |
| | TACCTGCAGATGAACAGCCTGAGGGCCGAGGACACCGCC GTGTACTACTGCGCCAGGTGGGTGTACGACCCCCTGTACG CCATGGACTACTGGGGCCAGGGCACCACCGTGACCGTGA GCAGC | |
| **2B3W-VL** | GACATCGTGCTGACCCAGACCCCCCTGAGCCTGAGCGTGA CCCCCGGCCAGCCCGCCAGCATCAGCTGCAGGGCCAGCG AGAGCGTGGACAACTACGGCATCAGCTTCATGAACTGGTT CCTGCAGAAGCCCGGCCAGCCCCCCCAGCTGCTGATCTAC GCCGCCAGCAACCAGGGCAGCGGCGTGCCCGACAGGTTC AGCGGCAGCGGCAGCGGCACCGACTTCACCCTGAAGATC AGCAGGGTGGAGGCCGAGGACGTGGGCGTGTACTACTGC CAGCAGAGCAAGGAGGTGCCCTGGACCTTCGGCCAGGGC ACCAAGGTGGAGATCAAG | 43 |
| **2B3R-VH** | GAGGTGCAGCTGGTGGAGAGCGGCGGCGGCCTGGTGCAG CCCGGCGGCAGCCTGAGGCTGAGCTGCGCCGCCAGCGGCT TCGTGTTCAGCAACTACGACATGAGCTGGGTGAGGCAGGC CCCCGGCAAGGGCCTGGAGTGGGTGGCCTACATCACCATC GGCGGCGGCACCACCTACTACCCCGACACCGTGAAGGGC AGGTTCACCATCAGCAGGGACAACGCCAAGAACAGCCTG TACCTGCAGATGAACAGCCTGAGGGCCGAGGACACCGCC GTGTACTACTGCGCCAGGAGGGTGTACGACCCCCTGTACG CCATGGACTACTGGGGCCAGGGCACCACCGTGACCGTGA GCAGC | 42 |
| **2B3R-VL** | GACATCGTGCTGACCCAGACCCCCCTGAGCCTGAGCGTGA CCCCCGGCCAGCCCGCCAGCATCAGCTGCAGGGCCAGCG AGAGCGTGGACAACTACGGCATCAGCTTCATGAACTGGTT CCTGCAGAAGCCCGGCCAGCCCCCCCAGCTGCTGATCTAC GCCGCCAGCAACCAGGGCAGCGGCGTGCCCGACAGGTTC AGCGGCAGCGGCAGCGGCACCGACTTCACCCTGAAGATC AGCAGGGTGGAGGCCGAGGACGTGGGCGTGTACTACTGC CAGCAGAGCAAGGAGGTGCCCTGGACCTTCGGCCAGGGC ACCAAGGTGGAGATCAAG | 43 |
| **7C5-VH** | GAGGTGCAGCTGGTGGAGAGCGGCGGCGGCCTGGTGAAG CCCGGCGGCAGCCTGAGGCTGAGCTGCGCCGCCAGCGGCT TCACCTTCAGCAGCTTCGGCATGAGCTGGGTGAGGCAGGC CCCCGGCAAGGGCCTGGAGCTGGTGGCCACCATCTACAGC AACGGCGACTACACCTACTACCCCGACAGCGTGAAGGGC | 44 |
| | AGGTTCACCGTGAGCAGGGACAACGCCAAGAACAGCCTG TACCTGCAGATGAACAGCCTGAGGGCCGAGGACACCGCC GTGTACTACTGCGCCAGGTACTACGGCAACTACGGCGGCT ACTTCGACTACTGGGGCCAGGGCACCCTGGTGACCGTGAG CAGC | |
| **7C5-VL** | GCCATCCAGATGACCCAGAGCCCCAGCAGCCTGAGCGCC AGCGTGGGCGACAGGGTGACCATCACCTGCAAGGCCAGC CAGAGCGTGAGCAACGACGTGGCCTGGTACCAGCAGAAG CCCGGCAAGGCCCCCAAGCTGCTGATCTACTACGCCAGCA ACAGGTACACCGGCGTGCCCAGCAGGTTCAGCGGCAGCG GCAGCGGCACCGACTTCACCCTGACCATCAGCAGCCTGCA GCCCGAGGACTTCGCCACCTACTACTGCCAGCAGGACTAC AGCAGCCCCCCCACCTTCGGCGGCGGCACCAAGGTGGAG ATCAAG | 45 |
| **8D4-VH** | CAGGTGCAGCTGGTGCAGAGCGGCGCCGAGGTGAAGAAG CCCGGCGCCAGCGTGAAGGTGAGCTGCAAGGCCAGCGGC TACACCTTCACCACCTACTGGATCCACTGGGTGAGGCAGG CCCCCGGCCAGGGCCTGGAGTGGATCGGCTACATCAACCC CACCACCGGCTACACCGAGTACAACCAGAAGTTCAAGGA CAGGGCCACCCTGACCGCCGACAAGAGCACCAGCACCGC CTACATGGAGCTGAGCAGCCTGAGGAGCGAGGACACCGC CGTGTACTACTGCGCCAGGGGCGTGAGGTACTACTTCGAC TACTGGGGCAAGGGCACCACCGTGACCGTGAGCAGC | 46 |
| **8D4-VL** | GACATCGTGATGACCCAGAGCCCCGACAGCCTGGCCGTGA GCCTGGGCGAGAGGGCCACCATCAACTGCAAGAGCAGCC AGAGCCTGCTGGACAGCAGGACCAGGAAGAACTACCTGG CCTGGTACCAGCAGAAGCCCGGCCAGCCCCCCAAGCTGCT GATCTACTGGGCCAGCACCAGGGAGAGCGGCGTGCCCGA CAGGTTCAGCGGCAGCGGCAGCGGCACCGACTTCACCCTG ACCATCAGCAGCCTGCAGGCCGAGGACGTGGCCGTGTACT ACTGCAAGCAGAGCTACAACCTGATCACCTTCGGCCAGGG CACCAAGCTGGAGATCAAG | 47 |

### Example 5. Property analysis

As illustrated in FIG. 7, three humanized antibodies were produced and purified using an Expi293 transient expression system, the quantitative binding affinity of each purified humanized antibody was analyzed by SDS-PAGE (7A) and ELISA, and a 2B3R clone thereof did not show the binding affinity. ELISA was performed in a manner similar to that of Example of FIG. 3, and antibodies were treated by 1/2 serial dilution from each concentration of 500 ng. The characteristics of purity and binding affinity of the humanized candidate antibodies were analyzed by SEC-HPLC, SPR (7B), and compared with a competitor Keytruda antibody as a positive control. In addition, each purified humanized antibody was bound to 293 cells expressing human PD-1 and analyzed by flow cytometry. As a result, the three humanized antibodies 2B3W, 7C5, and 8D4 showed high binding affinity, and then were selected as candidate antibodies for subsequent *in vivo,* anticancer efficacy analysis.

### Example 6. In vivo efficacy analysis of candidate antibodies

The present inventors analyzed the *in vivo* efficacy of the humanized antibodies 2B3W, 7C5, and 8D4 as candidate antibodies selected in Example 5 above.

This will be briefly described as follows. To analyze the *in vivo* efficacy of the humanized candidate antibodies, a tumor model was created using an MC38 cancer cell line (mouse colon cancer) expressing hPD-L1 in C57BL/6 knock-in mice expressing hPD-1 to measure markers for anticancer efficacy and hepatotoxicity in serum. Humanized candidate antibodies 2B3W, 7C5, and 8D4 were administered at a concentration of 10 mg/kg 5 times at 3-day intervals, and 5 mice per each humanized antibody were grouped.

As a result, as illustrated in FIG. 8A, when a tumor size was measured for each individual in each group, cancer growth was significantly inhibited in administration of all candidate antibodies, similarly to keytruda, a competitor antibody. An average value of the result values for each group was graphed. Intratumoral tumor infiltrated lymphocyte (TIL) analysis could not be performed because the tumor was almost removed.

In addition, as illustrated in FIG. 8B, at the end of the experiment, blood was collected from animals in each group and analyzed for hepatotoxicity, and no symptoms of hepatotoxicity were observed in the antibody-treated group of the present invention.

The above data confirmed that the humanized candidate antibodies 2B3W, 7C5, and 8D4 of the present invention not only exhibited excellent anticancer effects, but were also safe *in vivo* without hepatotoxicity.

### Example 7. Gene engineering evaluation of candidate antibodies

The present inventors performed gene engineering to increase the affinity to the antigen of the humanized antibodies 2B3W, 7C5, and 8D4 candidate antibodies selected in Example 5 above to obtain respective candidate antibodies. The characteristics of each antibody were compared with keytruda, a positive control, by SDS-PAGE, SEC-HPLC, and SPR analysis.

As a result, as illustrated in FIG. 9A, antibodies T14-01, T14-02, T14-03, T14-04, and T14-05 of 5 clones with improved affinity for 2B3W were obtained, and as compared with a positive control keytruda (9 nM), in the case of T14-05 (5.8 nM) with improved affinity from 2B3W, the affinity with antigen was higher than that of keytruda.

In addition, as illustrated in FIG. 9B, antibodies T15-01, T15-02, T15-03, T15-04, and T15-05 of 5 clones with improved affinity for 7C5 were obtained, and as compared to a positive control keytruda (9 nM), in the case of T15-01 (9.7 nM) with improved affinity from 7C5, it was shown that the affinity with antigen had the antigen binding affinity similar to that of keytruda.

On the other hand, as illustrated in FIG. 9C, antibodies T16-01 and T1-02 of two clones with improved affinity for 8D4 were obtained, and as compared to a positive control keytruda (9 nM), it was shown that the affinity with antigen was not improved.

Sequences of gene engineered antibodies of the humanized antibodies prepared as above were shown in Table 3 above.

In addition, as illustrated in FIG. 9D, T14-05 (5.8 nM) and T15-01 (9.7 nM) antibodies selected from candidate antibodies with improved affinity exhibited SEC-HPLC-purity and SPR-affinity as final compared with a competitor antibody Keytruda (9 nM).

Clones 2B3W-T14-05 (T14-05) and 7C5-T15-01 (T15-01), which were determined to have high affinity with antigen from the above results, were additionally selected.

### Example 8. Evaluation of binding affinity of candidate antibody to target cell

The present inventors analyzed the binding affinity of the humanized antibodies 2B3W, 7C5, 8D4, T14-05, and T15-01 selected in Examples above to hPD-1-overexpressing cells by flow cytometry.

As a result, as illustrated in FIG. 10A, all candidate antibodies showed excellent binding affinity to hPD-1 expressed on the cell surface.

In addition, as illustrated in FIG. 10B, when the binding affinity of the candidate antibodies was analyzed by flow cytometry on activated hCD8 T cells isolated from human blood, a proportional difference in binding affinity was shown for each clone, and antibodies T14-05 and T15-01 showed relatively high binding affinity.

### Example 9. PD-1/PD-L1 cell-based blockade assay evaluation and sequence analysis of candidate antibodies

The present inventors analyzed at a cell level whether the humanized antibodies 2B3W, 7C5, 8D4, T14-05, and T15-01 selected in Examples above inhibited the binding of effector cells expressing hPD-1 and APC cells expressing hPD-L1 as a receptor thereof.

The experiment was performed using a PD-1/PD-L1 Blockade Bioassay (Promega) kit, and when Jurkat effector cells expressing human PD-1 and having a luciferase reporter and CHO-K1 aAPC expressing human PD-L1 were co-cultured, a method was used to measure the expression level of luciferase by activating a TCR signal when the antibody was bound to hPD-1 to block the binding to hPD-L1 during treatment with the candidate antibody. Each candidate antibody was compared with candidate antibodies with improved affinity.

As a result, as illustrated in FIGS. 11A and 11B, it was confirmed that the PD-1 blocking effect of the candidate antibody against PD-L1 showed similar efficacy to that of keytruda, a positive control, in the T14-05 and T15-01 antibodies.

Accordingly, based on the results, clones T14-05 and T15-01 were selected as final candidate antibodies and sequenced. The results of analyzing the sequences of the clones T14-05 and T15-01 were shown in Table 3 above.
T14-05 (2B3W-T14-05) with improved affinity from 2B3W included a heavy chain variable region VH of SEQ ID NO: 48 and a light chain variable region VL of SEQ ID NO: 49; VH CDR1 was GFVFSNYD (SEQ ID NO: 1), VH CDR2 was ITIGGGTT (SEQ ID NO: 2), VH CDR3 was ARWRYDPLFAMDYW (SEQ ID NO: 56), VL CDR1 was ESVTDYGISFMN (SEQ ID NO: 57), VL CDR2 was AAS (SEQ ID NO: 6), and VL CDR3 was QQSKEVPWT (SEQ ID NO: 7); and
T15-01 (7C5-T15-01) with improved affinity from 7C5 included a heavy chain variable region VH of SEQ ID NO: 50 and a light chain variable region VL of SEQ ID NO: 51; VH CDR1 was GFTFSSFG (SEQ ID NO: 8), VH CDR2 was IYSNGDYT (SEQ ID NO: 9), VH CDR3 was ARYYGNYGGYFDY (SEQ ID NO: 10), VL CDR1 was QSVWDDLT (SEQ ID NO: 58), VL CDR2 was YAS (SEQ ID NO: 12), and VL CDR3 was QQDYSSPPT (SEQ ID NO: 13).

As illustrated in FIG. 12A, each CDR region was indicated in bold, and the sequence engineered for affinity improvement was underlined. The candidate antibody T14-05 had the same VH sequence as T14-04 with 2 amino acids changed in CDR3 of VH and had 2 amino acids changed in CDR1 of VL.

In addition, as illustrated in FIG. 12B, each CDR region was indicated in bold, and the sequence engineered for affinity improvement was underlined. The candidate antibody T15-01 had the same VH sequence of 7C5, but had four amino acids changed in CDR1 of VL.

In addition, as illustrated in FIG. 12C, each CDR region was indicated in bold, the sequence engineered for affinity improvement was underlined, and results of analyzing 8D4 and clones T16-01 and T16-02 sequences thereof were shown.

In addition, as illustrated in FIG. 12D, as a result of comparing the amino acid sequences of the finally selected T14-05 and T15-01 with an amino acid sequence of the positive control keytruda, new antibodies having different sequences including each CDR region were confirmed. In the antibody, a CPPCP amino acid sequence capable of increasing the stabilization of the antibody structure was used as a hinge portion of a human IgG4 isotype.

### Example 10. Interspecific cross-reactivity analysis of candidate antibodies

The present inventors analyzed the binding affinity of the humanized antibodies T14-05 and T15-01 finally selected in Examples above in human, monkey (cynomolgus, rhesus), canine, and mouse by ELISA using PD-1 antigens of each species.

As a result, as illustrated in FIG. 13, the two finally selected humanized candidate antibodies T14-05 and T15-01 had the cross-reactivity to monkey, but did not have the binding affinity to canine and mouse.

### Example 11. In vivo efficacy analysis of final candidate antibodies

As confirmed in Examples above, the present inventors analyzed the *in vivo* anticancer efficacy using T14-05 (5.8 nM), which was shown to have higher affinity than keytruda (9 nM), a positive control, as a representative antibody, even among humanized candidate antibodies with improved affinity.

For *in vivo* efficacy analysis, 1 × 10⁶ cells/mouse of a mouse colon cancer MC38 cell line expressing hPD-L1 was administered to C57BL/6 knock-in mice expressing hPD-1 to create a tumor model, and anticancer efficacy was analyzed. A T14-05 antibody (EU135-T14.05) was administered 6 times at three types of concentrations of 1 mg/kg, 3 mg/kg, and 5 mg/kg at 3-day intervals, and 6 animals were grouped for each antibody administered concentration. As a positive control, a competitor antibody, Keytruda, was administered at a concentration of 5 mg/kg and comparatively analyzed. Tumor size was measured twice a week from a first administration day. Each group was divided based on an average size in the tumor size of 100 to 150 mm³ of the mouse, and a number tag was created for each mouse to track the tumor size according to antibody administration.

As a result, as illustrated in FIG. 14A, when the tumor size was analyzed for each individual in each group, the tumor growth rate in all antibody-administered groups started to be delayed after second antibody administration, and after third antibody administration, it was observed that while the tumor size was reduced and necrosis began, cancer growth was significantly suppressed. These anticancer effects showed a difference in degree depending on the concentration of the antibody.

In addition, the average value of the tumor size result values in each group was graphed. As a result, as illustrated in FIG. 14B, compared to the continuous tumor growth of a control PBS, an antibody-administered group showed a decrease in cancer growth, and particularly, a 5 mg/kg concentration-administered group exhibited an anticancer effect better than that of a competitor antibody, keytruda. In addition, the T14-05-administered group showed anticancer efficacy depending on an antibody administration concentration.

In addition, after the third antibody administration, blood was collected from all individuals in each group, and the distribution rates of mouse CD4 and CD8 T cells were examined. 100 µl of blood collected from the eyehole was diluted at a ratio of 1 : 1 by adding 100 µl of PBS, and centrifuged on 100 µl Ficoll to isolate PBMCs by density gradient. The isolated PBMCs were analyzed by flow cytometry using mouse CD45, mouse CD8, and mouse CD4 antibodies.

As a result, as illustrated in FIG. 14C, when compared to a PBS-administered control group (G1), in all groups administered with the antibody, a ratio of mouse CD8 T cells for cancer cell death was increased, whereas a ratio of mouse CD4 T cells was decreased.

The result may mean that anticancer efficacy appears due to an increase in CD8 T cells, which are immune killer cells, in the antibody-administered group.

As described above, specific parts of the present disclosure have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. An antibody or an antigen-binding fragment thereof that specifically binds to programmed cell death protein 1 (PD-1), comprising:
a heavy chain CDR1 comprising at least one amino acid sequence selected from the group consisting of SEQ ID NOs: 1, 8, and 14;
a heavy chain CDR2 comprising at least one amino acid sequence selected from the group consisting of SEQ ID NOs: 2, 9, and 15;
a heavy chain CDR3 comprising at least one amino acid sequence selected from the group consisting of SEQ ID NOs: 3, 4, 10, 16, and 56;
a light chain CDR1 comprising at least one amino acid sequence selected from the group consisting of SEQ ID NOs: 5, 11, 17, 57, and 58;
a light chain CDR2 comprising at least one amino acid sequence selected from the group consisting of SEQ ID NOs: 6, 12, and 18; and
a light chain CDR3 comprising at least one amino acid sequence selected from the group consisting of SEQ ID NOs: 7, 13, and 19.

2. The antibody or the antigen-binding fragment thereof of claim 1, wherein the antibody or the antigen-binding fragment thereof comprises:
(i) a heavy chain variable region comprising a heavy chain CDR1 of SEQ ID NO: 1, a heavy chain CDR2 of SEQ ID NO: 2, and a heavy chain CDR3 of SEQ ID NO: 3, and a light chain variable region comprising a light chain CDR1 of SEQ ID NO: 5, a light chain CDR2 of SEQ ID NO: 6, and a light chain CDR3 of SEQ ID NO: 7;
(ii) a heavy chain variable region comprising a heavy chain CDR1 of SEQ ID NO: 1, a heavy chain CDR2 of SEQ ID NO: 2, and a heavy chain CDR3 of SEQ ID NO: 4, and a light chain variable region comprising a light chain CDR1 of SEQ ID NO: 5, a light chain CDR2 of SEQ ID NO: 6, and a light chain CDR3 of SEQ ID NO: 7;
(iii) a heavy chain variable region comprising a heavy chain CDR1 of SEQ ID NO: 8, a heavy chain CDR2 of SEQ ID NO: 9, and a heavy chain CDR3 of SEQ ID NO: 10, and a light chain variable region comprising a light chain CDR1 of SEQ ID NO: 11, a light chain CDR2 of SEQ ID NO: 12, and a light chain CDR3 of SEQ ID NO: 13;
(iv) a heavy chain variable region comprising a heavy chain CDR1 of SEQ ID NO: 14, a heavy chain CDR2 of SEQ ID NO: 15, and a heavy chain CDR3 of SEQ ID NO: 16, and a light chain variable region comprising a light chain CDR1 of SEQ ID NO: 17, a light chain CDR2 of SEQ ID NO: 18, and a light chain CDR3 of SEQ ID NO: 19;
(v) a heavy chain variable region comprising a heavy chain CDR1 of SEQ ID NO: 1, a heavy chain CDR2 of SEQ ID NO: 2, and a heavy chain CDR3 of SEQ ID NO: 56, and a light chain variable region comprising a light chain CDR1 of SEQ ID NO: 57, a light chain CDR2 of SEQ ID NO: 6, and a light chain CDR3 of SEQ ID NO: 7; or
(vi) a heavy chain variable region comprising a heavy chain CDR1 of SEQ ID NO: 8, a heavy chain CDR2 of SEQ ID NO: 9, and a heavy chain CDR3 of SEQ ID NO: 10, and a light chain variable region including a light chain CDR1 of SEQ ID NO: 58, a light chain CDR2 of SEQ ID NO: 12, and a light chain CDR3 of SEQ ID NO: 13.

3. The antibody or the antigen-binding fragment thereof of claim 1, wherein the antibody or the antigen-binding fragment thereof comprises a heavy chain variable region comprising at least one amino acid sequence selected from the group consisting of SEQ ID NOs: 20, 21, 23, 25, 27, 28, 30, 32, 48, and 50.

4. The antibody or the antigen-binding fragment thereof of claim 1, wherein the antibody or the antigen-binding fragment thereof comprises a light chain variable region comprising at least one amino acid sequence selected from the group consisting of SEQ ID NOs: 22, 24, 26, 29, 31, 33, 49, 51, 54, and 55.

5. The antibody or the antigen-binding fragment thereof of claim 1, wherein the antibody or the antigen-binding fragment thereof comprises:
a heavy chain variable region of SEQ ID NO: 20 and a light chain variable region of SEQ ID NO: 22;
a heavy chain variable region of SEQ ID NO: 21 and a light chain variable region of SEQ ID NO: 22;
a heavy chain variable region of SEQ ID NO: 23 and a light chain variable region of SEQ ID NO: 24;
a heavy chain variable region of SEQ ID NO: 25 and a light chain variable region of SEQ ID NO: 26;
a heavy chain variable region of SEQ ID NO: 27 and a light chain variable region of SEQ ID NO: 29;
a heavy chain variable region of SEQ ID NO: 28 and a light chain variable region of SEQ ID NO: 29;
a heavy chain variable region of SEQ ID NO: 30 and a light chain variable region of SEQ ID NO: 31;
a heavy chain variable region of SEQ ID NO: 32 and a light chain variable region of SEQ ID NO: 33;
a heavy chain variable region of SEQ ID NO: 48 and a light chain variable region of SEQ ID NO: 49;
a heavy chain variable region of SEQ ID NO: 50 and a light chain variable region of SEQ ID NO: 51;
a heavy chain variable region of SEQ ID NO: 52 and a light chain variable region of SEQ ID NO: 53; or
a heavy chain variable region of SEQ ID NO: 54 and a light chain variable region of SEQ ID NO: 55.

6. The antibody or the antigen-binding fragment thereof of claim 1, wherein the antibody or the antigen-binding fragment thereof comprises single-chain Fvs (scFv), single-chain antibody, Fab, F(ab'), and disulfide-bonded Fvs (sdFv).

7. The antibody or the antigen-binding fragment thereof of claim 6, wherein the scFv has a heavy chain variable region and a light chain variable region which are linked to each other via a linker.

8. A nucleic acid encoding the antibody or the antigen-binding fragment thereof according to any one of claims 1 to 7.

9. A recombinant expression vector comprising the nucleic acid of claim 8.

10. Host cells transfected with the recombinant expression vector of claim 9.

11. The host cells of claim 10, wherein the host cells comprise COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, or HT1080.

12. A method for preparing an antibody or an antigen-binding fragment thereof specifically binding to PD-1, comprising culturing the host cells of claim 10 to produce antibodies; and isolating and purifying the produced antibodies.

13. A bispecific or multispecific antibody comprising the antibody or the antigen-binding fragment thereof according to any one of claims 1 to 7.

14. The bispecific or multispecific antibody of claim 13, wherein the bispecific or multispecific antibody comprises, as a partner, an antibody or an antigen-binding fragment thereof binding to at least one selected from the group consisting of FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

15. An immune cell engaging bispecific or multispecific antibody comprising scFv of the antibody according to any one of claims 1 to 7 and at least one of scFv of an antibody binding to an immune cell activating antigen.

16. The bispecific or multispecific antibody of claim 15, wherein the immune cell activating antigen comprises:
a T cell activating antigen such as CD3, TCRα, TCRβ, TCRγ, TCRξ, ICOS, CD28, CD27, HVEM, LIGHT, CD40, 4-1BB, OX40, DR3, GITR, CD30, TIM1, SLAM, CD2, or CD226;
an NK cell activating antigen such as NKp30, NKp40, NKp44, NKp46, NKG2D, DNAM1, DAP10, CD16 (CD16a or CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160;
a B-cell activating antigen such as OX40, CD40, or CD70;
a macrophage activating antigen such as CD2 agonist, CD40, CD70, Toll-like Receptor (TCR) agonist, CD47, STING, or OX40L; or
a dendritic cell activating antigen such as a CD2 agonist, OX40, OX40L, 41BB agonist, TCR agonist, CD47 agonist, or STING agonist.

17. An antibody-drug conjugate (ADC) in which the antibody or the antigen-binding fragment thereof according to any one of claims 1 to 7 binds to a drug.

18. The antibody-drug conjugate of claim 17, wherein the drug is at least one selected from the group consisting of maytansinoid, auristatin (including MMAE and MMAF), aminopterin, actinomycin, bleomycin, thalisomycin, camptothecin, N8-acetyl spermidine, 1-(2 chloroethyl)-1,2-dimethyl sulfonyl hydrazide, esperamycin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocamycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, bischloroethylnitrosourea (CNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, 5-ethynyl-1-beta-dribofuranosylimidazole-4-carboxamide (EICAR), hydroxyurea, deferoxamine, fluxuridine, doxifluridine, raltitrexed, cytarabine(ara C), cytosine arabinoside, fludarabine, tamoxifen, raloxifene, megestrol, goserelin, leuprolide acetate, flutamide, bicalutamide, EB 1089, CB1093, KH1060, verteporfin, phthalocyanine, photosensitizer Pe4, demethoxy-hypocrellin A, interferon-α, interferon-y, tumor necrosis factor, gemcitabine, velcade, revamid, thalamid, lovastatin, 1-methyl-4-phenylpyridiniumion, staurosporine, actinomycin D, dactinomycin, bleomycin A2, bleomycin B2, peplomycin, epirubicin, pirarubicin, zorubicin, mitoxantrone, verapamil, thapsigargin, nuclease, and toxins derived from bacteria, animals, and plants.

19. The antibody-drug conjugate of claim 17, wherein the antibody or the antigen-binding fragment thereof binds to a drug via a linker.

20. The antibody-drug conjugate of claim 19, wherein the linker is a cleavable linker or a non-cleavable linker.

21. The antibody-drug conjugate of claim 20, wherein the cleavable linker is an acid-labile linker, a disulfide linker, a peptide linker, or a beta-glucuronide linker, or the non-cleavable linker includes a thioether group or a maleimidocaproyl group.

22. The antibody-drug conjugate of claim 19, wherein the linker is bound to cysteine residues exposed during reduction of disulfide bonds of the antibody or to cysteine residues present in a tag binding to the antibody.

23. A chimeric antigen receptor (CAR) comprising an extracellular domain including an antigen-binding site, a transmembrane domain, and an intracellular signaling domain,
wherein the antigen-binding site of the extracellular domain is scFv of the antibody according to any one of claims 1 to 7.

24. Immune cells into which the chimeric antigen receptor (CAR) according to claim 23 is introduced.

25. The immune cells of claim 24, wherein the immune cells are one or more selected from the group consisting of T cells, NK cells, cytokine induced killer cells (CIKs), activated cytotoxic T lymphocytes (CTLs), macrophages, tumor-infiltrating lymphocytes (TILs), and dendritic cells.

26. The immune cells of claim 23, wherein the scFv for the antibody other than an anti-PD-1 antibody is additionally introduced with the chimeric antigen receptor (CAR) included as the antigen-binding site of the extracellular domain.

27. The immune cells of claim 26, wherein the antibody other than the anti-PD-1 antibody is an antibody or an antigen-binding fragment thereof targeting at least one selected from the group consisting of FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

28. A composition for combination therapy comprising the immune cells of claim 24 and a drug other than an anti-PD-1 antibody.

29. The composition for combination therapy of claim 28, wherein the drug other than the anti-PD-1 antibody is an antibody or an antigen-binding fragment thereof targeting at least one selected from the group consisting of FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

30. A composition for combination therapy comprising the antibody or the antigen-binding fragment thereof according to any one of claims 1 to 7 and at least one selected from the group consisting of:
(i) immune cells;
(ii) immune cells comprising an antigen receptor (CAR) containing scFv for an antibody other than an anti-PD-1 antibody as an extracellular domain; and
(iii) an immune checkpoint inhibitor.

31. The composition of claim 30, wherein the immune cells are one or more selected from the group consisting of T cells, NK cells, cytokine induced killer cells (CIKs), activated cytotoxic T lymphocytes (CTLs), macrophages, tumor-infiltrating lymphocytes (TILs), and dendritic cells.

32. The composition of claim 30, wherein the antibody other than the anti-PD-1 antibody is an antibody or an antigen-binding fragment thereof targeting at least one selected from the group consisting of FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

33. The composition of claim 30, wherein the immune checkpoint inhibitor is a drug targeting TIGIT, PD-L1, BTLA, CTLA-4, VISTA, LAG3, TIM3, CD137(4-1BB), CD258(LIGHT), MARCO, CD134(OX40), CD28, CD278(ICOS), CD27, CD154(CD40L), CD357(GITR), CD30, DR3, CD226(DNAM1), CD96, CD200, or CD200R.

34. A composition for combination therapy comprising the bispecific or multispecific antibody according to claim 13 and at least one selected from the group consisting of:
(i) immune cells;
(ii) immune cells comprising an antigen receptor (CAR) containing an scFv fragment for an antibody other than an anti-PD-1 antibody as an extracellular domain; and
(iii) an immune checkpoint inhibitor.

35. The composition of claim 34, wherein the antibody other than the anti-PD-1 antibody is an antibody or an antigen-binding fragment thereof targeting at least one selected from the group consisting of FGFR3, TIGIT, PD-L1, BTLA, CTLA-4, VISTA, LAG3, TIM3, CD137(4-1BB), VISTA, CD258(LIGHT), MARCO, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, and NRP1.

36. The composition of claim 34, wherein the immune checkpoint inhibitor is a drug targeting TIGIT, PD-L1, BTLA, CTLA-4, VISTA, LAG3, TIM3, CD137(4-1BB), CD258(LIGHT), MARCO, CD134(OX40), CD28, CD278(ICOS), CD27, CD154(CD40L), CD357(GITR), CD30, DR3, CD226(DNAM1), CD96, CD200, or CD200R.

37. A composition for treating cancer comprising an antibody or an antigen-binding fragment thereof according to any one of claims 1 to 7, a bispecific or multispecific antibody comprising the antibody or the antigen-binding fragment thereof, an antibody-drug conjugate comprising the antibody or the antigen-binding fragment thereof, a chimeric antigen receptor comprising the antibody or the antigen-binding fragment thereof, or immune cells comprising the chimeric antigen receptor.
